# EUROPEAN PATENT APPLICATION

(11) **EP 2 495 310 A2**
(43) Date of publication of application: **05.09.2012**
(21) Application number: 12158304.1
(22) Date of filing: 24.05.2006
(51) Int. Cl.: C12N 5/071

(54) **Microscale micropatterned engineered in vitro tissue**

(30) Priority: 24.05.2005 US 684508 P
(62) Divisional of application: 06760327.4
(71) Applicant: The Regents Of the University of California, Oakland, CA 94607-5200 (US)
(72) Inventor: Bhatia, Sangeeta N., Lexington, MA 02420 (US); Khetani, Salman R., Quincy, MA 02169 (US)
(74) Representative: Cornish, Kristina Victoria Joy

(57) **Abstract**

The disclosure provides *an in vitro* culture systems. The invention provides methods and systems useful for developing and *in vitro* engineered tissue, method of using the tissue and compositions comprising the tissue.

## Description

### CROSS REFERENCE TO RELATED APPLICATIONS

This application claims priority under 35 U.S.C. §119 to U.S. Provisional Application Serial No. 60/684,508, filed May 24, 2005, the disclosure of which is incorporated herein by reference in its entirety.

The disclosures of U.S. Provisional Application Serial No. 60/450,532, filed February 26, 2003; International Patent Application No. PCT/US2004/006018; U.S. Provisional Application Serial No. 60/302,879, filed July 3, 2001; and International Patent Application No. PCT/US2002/21207, are also incorporated herein by reference.

### TECHNICAL FIELD

The disclosure relates to tissue compositions, methods and apparati for culturing tissue. More particularly, the disclosure relates to micropatterned cellular tissue capable of growing and sustaining a desired function in culture.

### BACKGROUND

Historically cell culture techniques and tissue development failed to take into account the necessary microenvironment for cell-cell and cell-matrix communication as well as an adequate diffusional environment for delivery of nutrients and removal of waste products. Cell culture techniques and understanding of the complex interactions cells have with one another and the surrounding environment have improved in the past decade.

While many methods and bioreactors have been developed to grow tissue for the purposes of generating artificial tissues for transplantation or for toxicology studies, these bioreactors do not adequately simulate, in vitro, the mechanisms by which nutrients, gases, and cell-cell interactions are delivered and performed in vivo. For example, cells in living tissue are "polarized" with respect to diffusion gradients. Differential delivery of oxygen and nutrients, as occurs *in* vivo by means of the capillary system, controls the relative functions of tissue cells and their maturation. Thus, cell culture systems and bioreactors that do not simulate these in vivo delivery mechanisms do not provide a sufficient corollary to in *vivo* environments to develop tissues or measure tissue responses in *vitro.*

The ability to develop in vitro tissue, such as hepatic tissue, can provide a supply of tissue for toxicology testing, extracorporeal liver devices as well as tissue for transplantation. For example, liver failure is the cause of death of over 30,000 patients in the United States every year and over 2 million patients worldwide. Current treatments are largely palliative- including delivery of fluids and serum proteins. The only therapy proven to alter mortality is orthotopic liver transplants; however, organs are in scarce supply (McGuire et al., Dig Dis. 13(6):379-88 (1995)).

Cell-based therapies have been proposed as an alternative to whole organ transplantation, a temporary bridge to transplantation, and/or an adjunct to traditional therapies during liver recovery. Three main approaches have been proposed: transplantation of isolated hepatocytes via injection into the blood stream, development and implantation of hepatocellular tissue constructs, and perfusion of blood through an extracorporeal circuit containing hepatocytes. Investigation in all, three areas has dramatically increased in the last decade, yet progress has been stymied by the propensity for isolated hepatocytes to rapidly lose many key liver-specific functions.

Drug-induced liver disease represents a major economic challenge for the pharmaceutical industry since unforeseen liver toxicity and poor bioavailability issues cause more than 50% of new drug candidates to fail in Phase I clinical trials. Also, a third of drug withdrawals from the market and more than half of all warning labels on approved drugs are primarily due to adverse affects on the liver. Therefore, besides pharmacological properties, ADME/Tox (absorption, distribution, metabolism, excretion and toxicity) characteristics are crucial determinants of the ultimate clinical success of a drug. This realization has led to an early introduction of ADME/Tox screening during the drug discovery process, in an effort to select against drugs with problematic properties.

Animal models provide a limited view of human toxicity due to species-specific variations as well as animal-to-animal variability, necessitating 5-10 animals per compound per dase, sometimes in both genders. Incorporating in vitro models into drug development provides several advantages: earlier elimination of problematic drugs, reduction in variability by allowing hundreds of experiments per animal and human models without patient exposure. In the case of the liver, in vitro models can provide valuable information on drug uptake and metabolism, enzyme induction, and drug-drug interactions affecting metabolism and hepatotoxicity.

Several *in vitro* liver models are used for short-term (hours) investigation of xenobiotic metabolism and toxicity. Perfused whole organs, liver slices and wedge biopsies maintain many aspects of liver's *in vivo* microenvironment; however, such systems suffer from limited drug availability to inner cell layers, limited viability (< 24h) and are not suitable for enzyme induction studies. Isolated liver microsomes, which are cellular fragments that contain mostly CYP450 enzymes, are used primarily to investigate drug metabolism via the phase I pathways (oxidation, reduction, hydrolysis and the like). However, microsomes lack many important aspects of the cellular machinery where dynamic changes occur (*i.e*. gene expression, protein synthesis) to alter drug metabolism, toxicity and drug-drug interactions. Besides microsomes, cell lines derived from hepatoblastomas (HepG2) or from immortalization of primary hepatocytes (HepLiu, SV40 immortalized) are finding limited use as reproducible, inexpensive models of hepatic tissue. However, no cell line has been developed to date that maintains physiologic levels of liver-specific functions. Usually such cell lines are plagued by an abnormal repertoire of hepatic functions.

Current in vitro liver models used by the pharmaceutical industry, though useful in a limited capacity, are not fully predictive of in *vivo* liver metabolism and toxicity. Thus, research has increasingly turned towards using isolated primary human hepatocytes as the gold standard for in vitro studies; however, hepatocytes are notoriously difficult to maintain in culture as they rapidly lose viability and phenotypic functions.

### SUMMARY

The invention provides methods, systems, and composition that overcome the limitations of current techniques. The invention provides an engineered in vitro model of parenchymal tissue (e.g., human liver) that remains optimally functional for several weeks. More specifically, the invention utilized microfabrication techniques to create 2-D and 3-D cultures that comprise parenchymal cells (*e.g*., primary human hepatocytes) spatially arranged in a bounded geometry by non-parenchymal cells in a micropatterned coculture. The bounded geometry may be of any regular or irregular dimension (e.g., circular, semi-circular, spheroidal islands of a pre-defined diameter, length, width etc., typically about 250-750 µm). For example, the invention demonstrates that micropatterned human cocultures reproducibly out perform (by several fold) their randomly distributed counterparts, which contain similar cell ratios and numbers. The invention demonstrates that co-cultures require an optimal balance of homotypic and heterotypic interaction to function optimally.

The invention provides an in vitro cellular composition, comprising one or more populations of parenchymal cells defining a cellular island; and a population of non-parenchymal cells, wherein the non-parenchymal cells define a geometric border of the cellular island.

The invention further provides a method of making a plurality of cellular islands on a substrate. The method comprises spotting an adherence material on a substrate at spatially different locations each spot having a defined geometric size and/or shape; contracting the substrate with a population of cells that selectively adhere to the adherence material and/or substrate; and culturing the cells on the substrate to generate a plurality of cellular islands.

The invention also provides an assay system comprising contacting an artificial tissue the tissue comprising parenchymal cells having a bounded geometry bordered by non-parenchymal cells wherein the bounded geometry has at least one dimension from side to side of the bounded geometry of about 250µm to 150µm; contacting the artificial tissue with a test agent; and measuring an activity selected from gene expression, cell function, metabolic activity, morphology, and a combination thereof, of the artificial tissue.

The invention provides an artificial tissue comprising islands of parenchymal cells surrounded by stromal cells wherein the islands of parenchymal cells are about 250µm to 750µm in diameter or width.

The invention further provides a method of producing a tissue *in vitro.* The method comprising seeding a first population of cells on a substrate having defined regions for attachment of the first population of cells, wherein the defined regions comprise a bounded geometric dimension of about 250µm to 750µm; seeding a second population of cells on the substrate, such that the second population of cells surround or adhere adjacent to the first population of cells; and culturing the cells under conditions and for a sufficient period of time to generate a tissue.

The details of one or more embodiments of the disclosure are set forth in the accompanying drawings and the description below . Other features, objects, and advantages will be apparent from the description and drawings, and from the claims.

### DESCRIPTION OF DRAWINGS

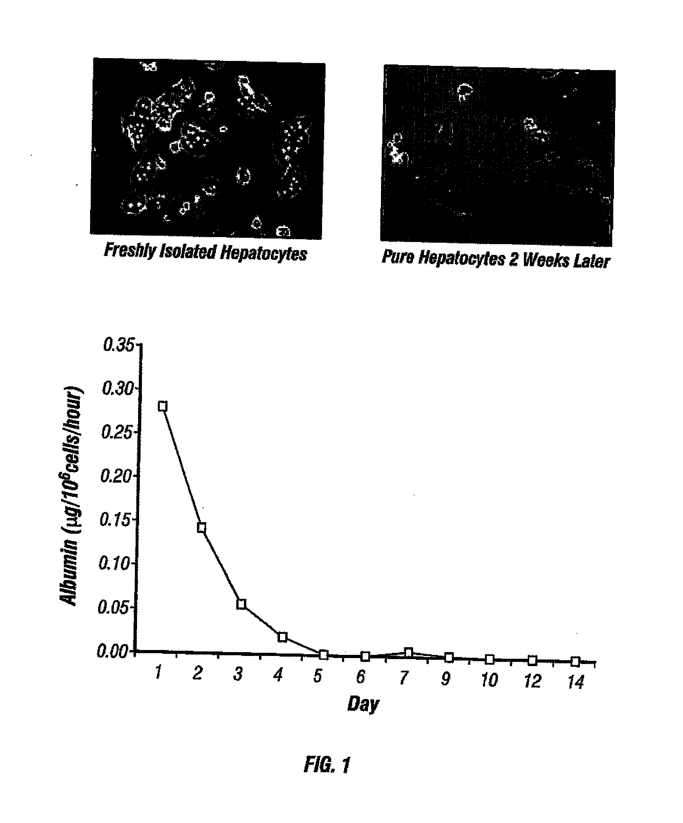

Figure 1 shows that upon isolation from their in vivo microenvironment, hepatocyte rapidly lose viability and important liver-specific functions such as albumin secretion, urea synthesis and cytochrome P450 activity. After about a week in culture on collagen-coated dishes, hepatocytes show a fibroblastic morphology. Freshly isolated hepatocytes, on the other hand, show a polygonal morphology with distinct nuclei and nucleoli and bright intercellular boundaries (bile canaliculi).

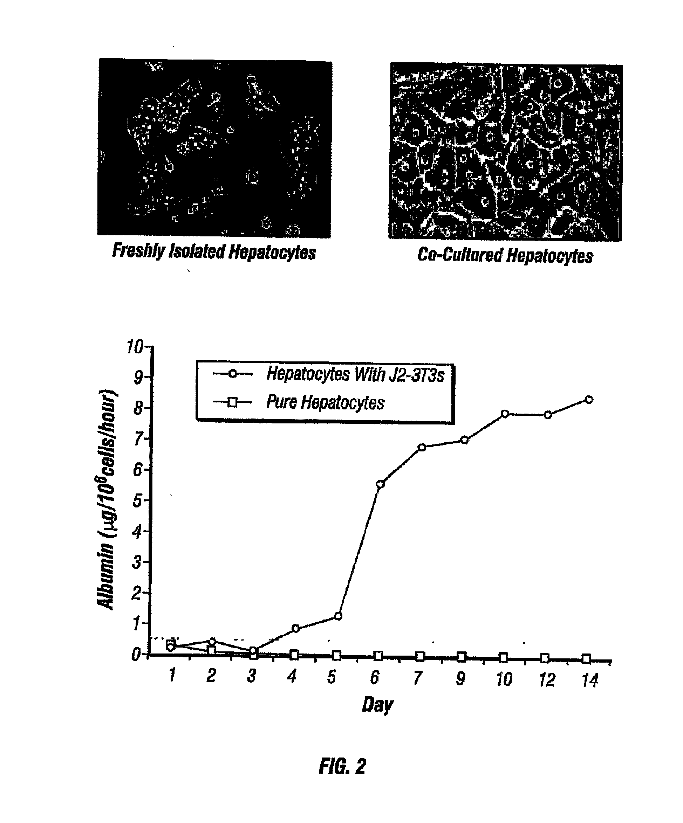

Figure 2 shows co-cultivation of hepatocytes with J2-3T3 fibroblasts on collagen-coated surfaces. Hepatic functions such as albumin secretion, (as well as P450 activity) are upregulated in co-culture, whereas in pure culture, they decline and hepatocytes lose viability. Functionally stable hepatocytes in co-culture maintain the polygonal morphology, distinct nuclei and nucleoli, and visible bile-canaliculi seen typically in freshly isolated hepatocytes.

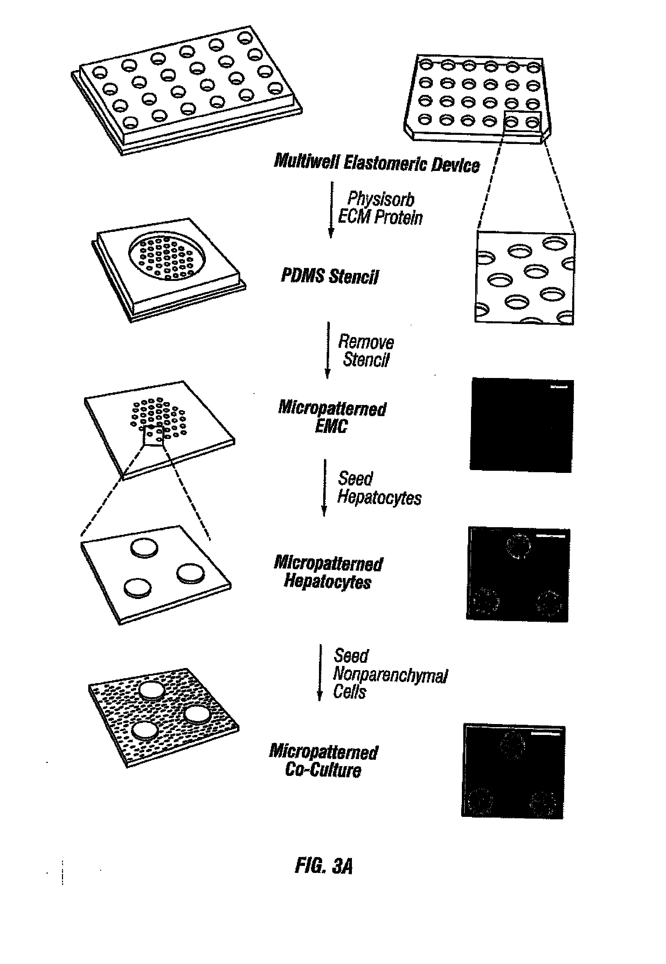

Figure 3A-C shows a soft lithographic process to fabricate microscale liver tissues in a multiwell format. A) Schematic of the process flow aside photomicrographs taken at each step. A reusable PDMS stencil is seen consisting of membranes with through-holes at the bottom of each well in a 24-well mold. To micropattern all wells simultaneously, the device is sealed under dry conditions to a culture substrate. A photograph of a device (scale bar represents 2 cm) sealed to a polystyrene omni-tray is seen along with an electron micrograph of a thin stencil membrane. Each well is incubated with a solution of extracellular matrix protein to allow protein to adsorb to the substrate via the through-holes. The stencil is then peeled off leaving micropatterned ECM protein on the substrate (fluorescently labeled collagen pattern shown). A 24-well PDMS 'blank' lacking membranes is then sealed to the plate before cell seeding. Primary hepatocytes selectively adhere to matrix-coated domains, allowing supportive non-parenchymal cells to be seeded in serum-supplemented culture media into the remaining bare areas hepatocytes labeled green and fibroblasts orange, scale bar is 500 µm). B) Photograph of a 24-well device with repeating hepatic microstructures (37 colonies of 500µm diameter in each well) stained purple by MTT (scale bar 2 cm and 1 cm for enlargement). C) Phase contrast micrographs of optimal micropatterned human co-cultures. Primary human hepatocytes are spatially arranged in -500 µm collagen coated islands with -1200 µm center-to-center spacing, surrounded by murine embryonic 3T3-J2 fibroblasts. Images depict pattern fidelity over time and hepatocellular morphologic features include bile canaliculi (scale bars are 500µm, 500 µm, and 100 µm from left to right).

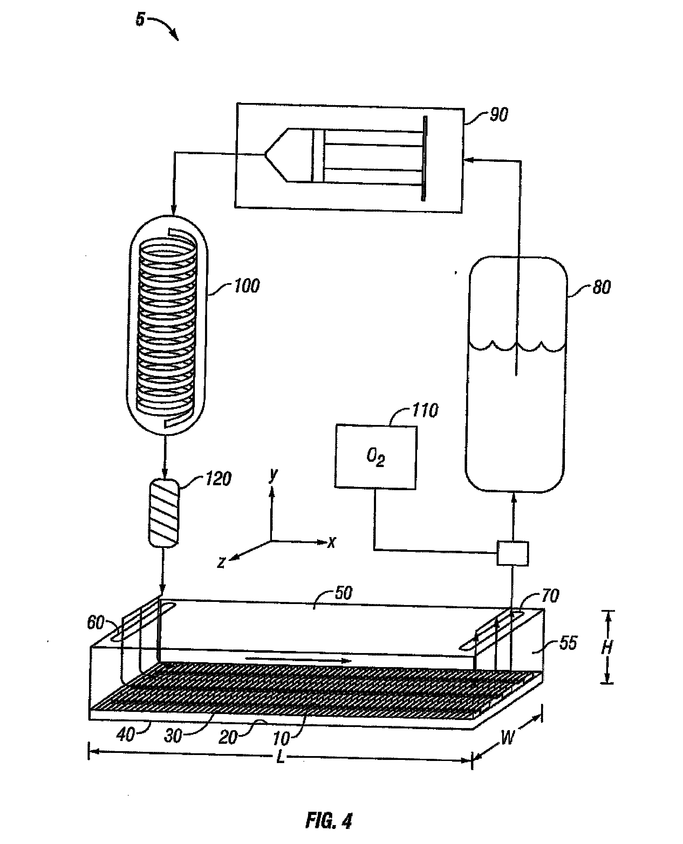

Figure 4 is a schematic showing a bioreactor system of the disclosure.

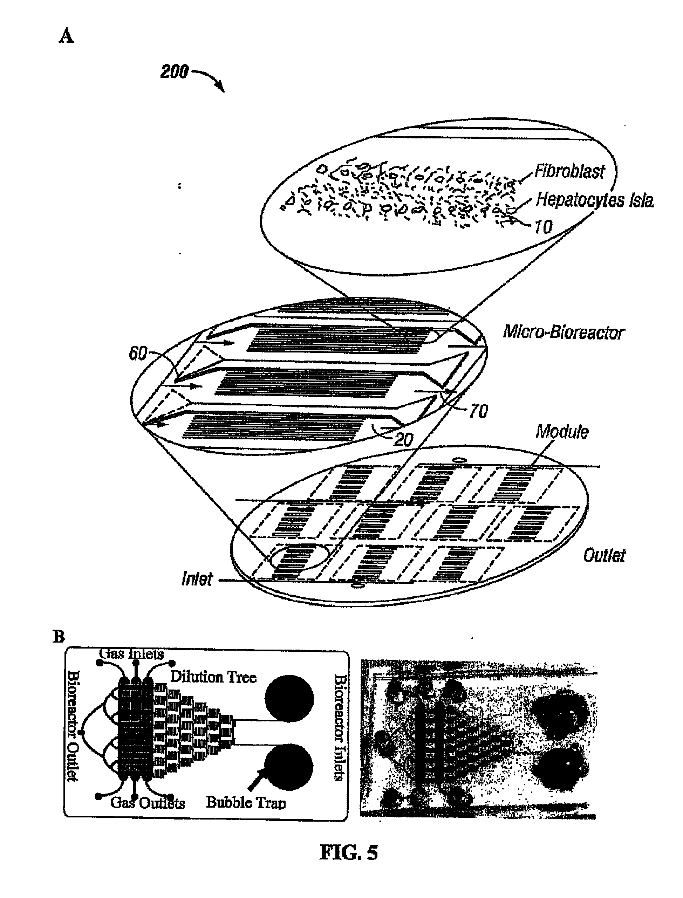

Figure 5A-B is a schematic of a high-throughput, micro-bioreactor array. (A) Bottom panel depicts array of 50 micro-bioreactors in ten modules of 5 micro-bioreactors each. Modules are laid out on a 4-inch glass wafer with 2 alignment holes. Reactors are formed by an underlying glass surface that is micropatterned with collagen and a silicone "lid" that confines the flow of perfusate. Each module has a single inlet and single outlet. Middle panel depicts 3 of the 5 micro-bioreactors in a module with a common inlet and outlet. Top panel depicts micropatterned co-cultures with aligned hepatocytes and fibroblasts in each micro-bioreactor. (B) shows a further schematic and embodiments of a bioreactors.

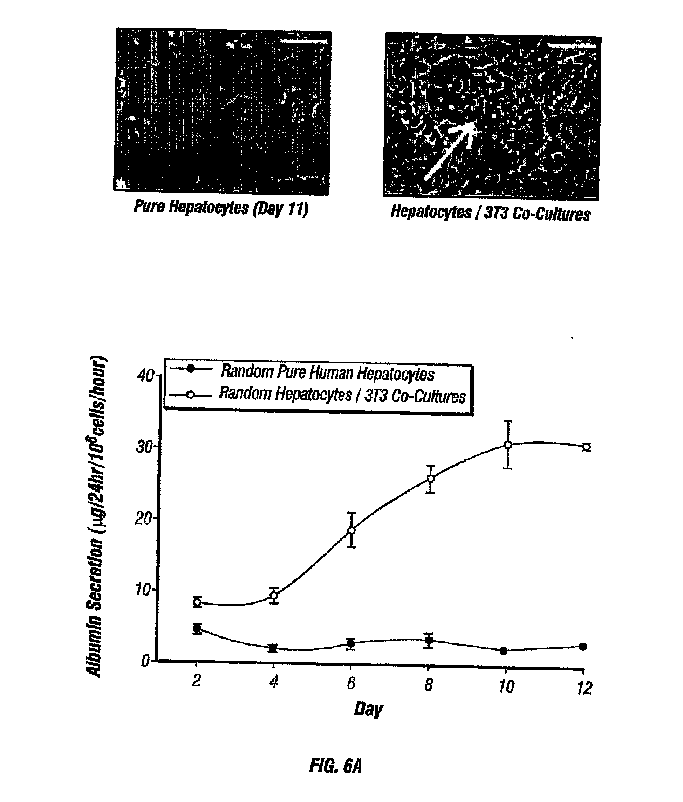

Figure 6A-C shows functional optimization of human hepatocyte cultures and co-cultures via micropatterning. A) Rate of albumin secretion (a marker for synthesis of liver-specific proteins) by human hepatocytes in pure monolayer cultures and upon co-cultivation with 3T3-J2 murine embryonic fibroblasts randomly distributed on collagen-coated polystyrene . Several other functions were also stabilized in hepatocyte/3T3 cocultures (i.e. urea secretion, cytochrome-P450 activity) as compared to unstable pure monolayer. In pure cultures, hepatocytes) adopt a 'fibroblastic' morphology, whereas in co-cultures they maintain their polygonal shape (arrow), distinct nuclei, and visible bile canaliculi as typically seen *in vivo* (scale bars represent 100µm). B) Functional optimization of human hepatocyte/3T3 co-cultures using microfabrication techniques. Primary human hepatocytes were spatially organized on collagen-coated islands of prescribed dimensions using photolithography, and then surrounded by 3T3-J2. fibroblasts 24 hours after hepatocyte attachment and spreading. Island size (36, 490, 4800µm) and center-to-center spacing (e.g. 90µm for 36µm islands) between islands for each configuration were selected to keep total cell numbers and ratios of two cell types constant. Dimensions were also chosen to enable comparisons with previous work using primary rat hepatocytes. Randomly distributed control co-cultures ('Random') on collagen were also generated to enable comparisons. Cumulative liver-specific functions over 2 weeks are compared for micropatterned human hepatocyte/3T3 co-cultures. C) Hepatocytes were micropatterned but not surrounded by fibroblasts in 'pure micropatterned cultures'. Liver-specific functions were compared. Error bars represent standard error of the mean (n - 3).

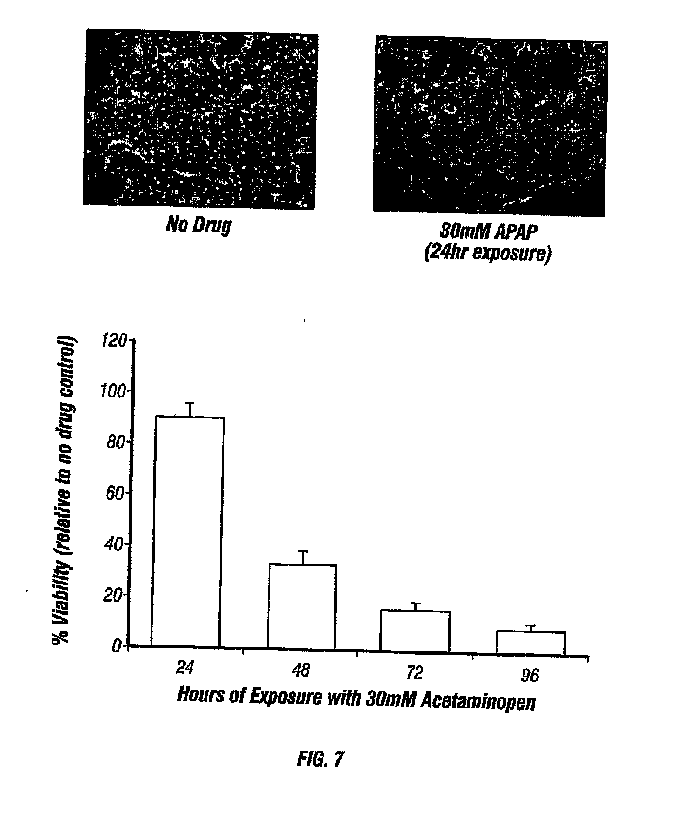

Figure 7 shows chronic toxicity testing with micropatterned human cocultures. Loss of viability in cocultures upon incubation with acetaminophen (30mM) for increasing time intervals. Micrographs shown depict hepatocyte morphology with or without drug.

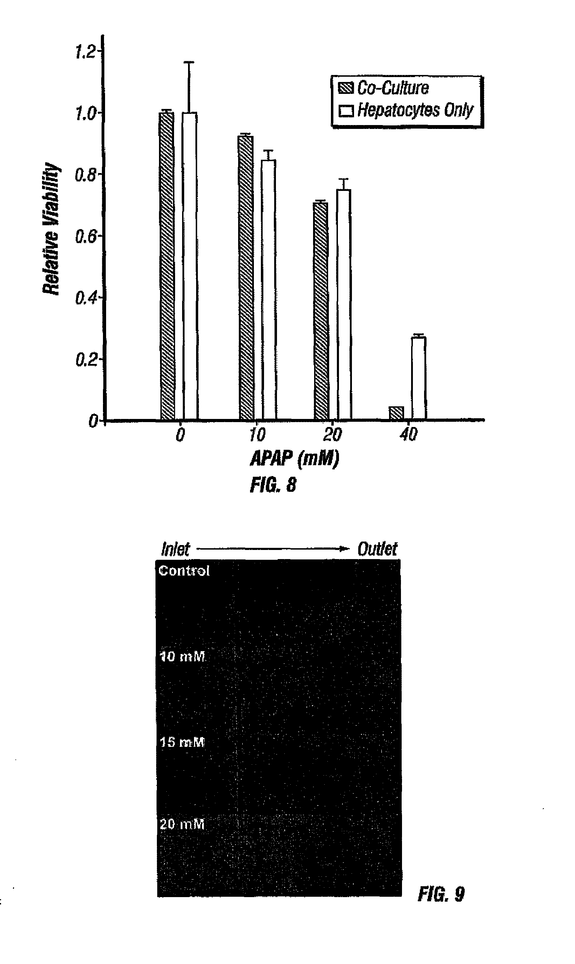

Figure 8 shows the viability of co-cultures and hepatocyte-only cultures as assessed by MTT after 24-hour exposure to varying concentrations of APAP.

Figure 9 shows photomicrograph of cultures stained with MTT after 24 hours perfusion with indicated concentrations of APAP.

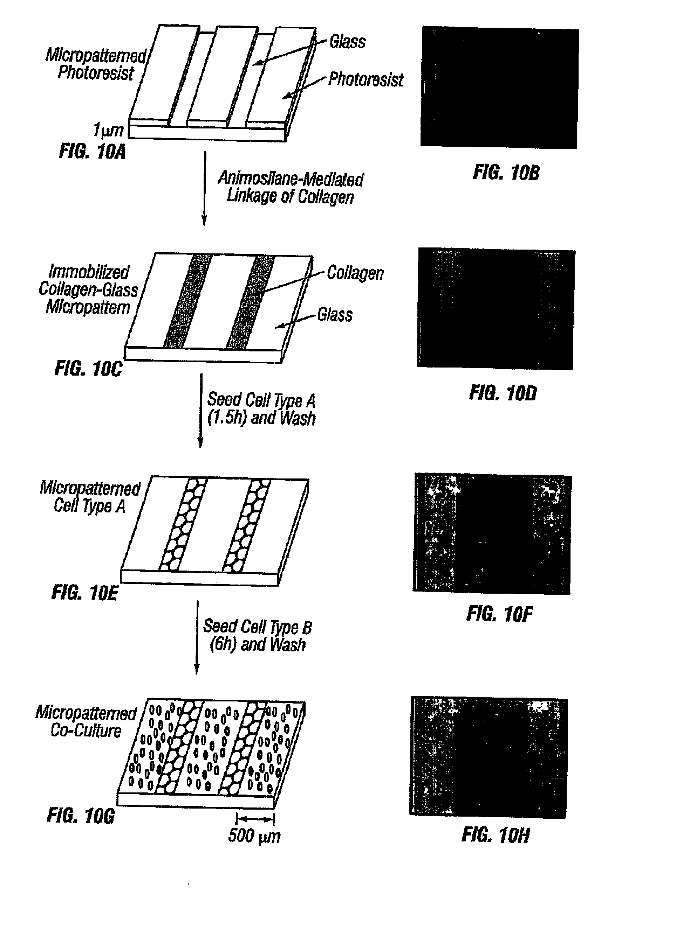

Figure 10A-H is a schematic of method for generating micropatterned co-cultures. Briefly, photolithography is used to pattern photoresist on glass substrates (A). A fluorescent micrograph of the photoresist pattern is shown in 'B'. After letting collagen adsorb to the entire wafer, the photoresist is stripped off using acetone, leaving collagen patterns on glass (C-D). The substrate is then coated with bovine serum albumin to prevent non-specific cell attachment to regions without collagen. Hepatocytes are seeded at high density in serum-free medium (-1 million cells per 35mm wafer) several times to ensure near complete coverage of collagen areas, without significant nonspecific attachment (E-F). One to two hours after attachment, floating cells are washed away. Next day, after the hepatocytes have spread out to fill the patterns, fibroblasts are seeded in serum-supplemented medium (G-H).

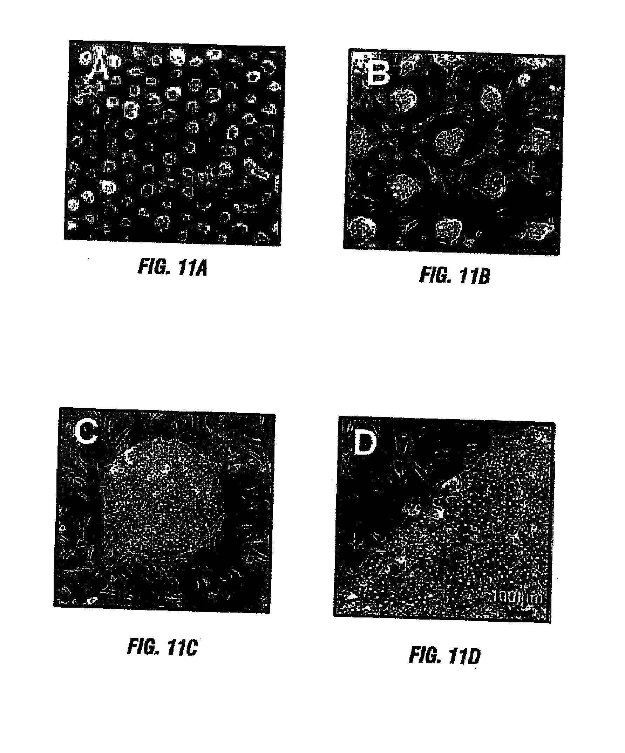

Figure 11 shows micropatterned rat cocultures, with constant ratio of cell populations, as well as constant cell numbers. Phase contrast micrographs of micropatterned cocultures indicate broad range of heterotypic interface achieved despite similar cellular constituents.

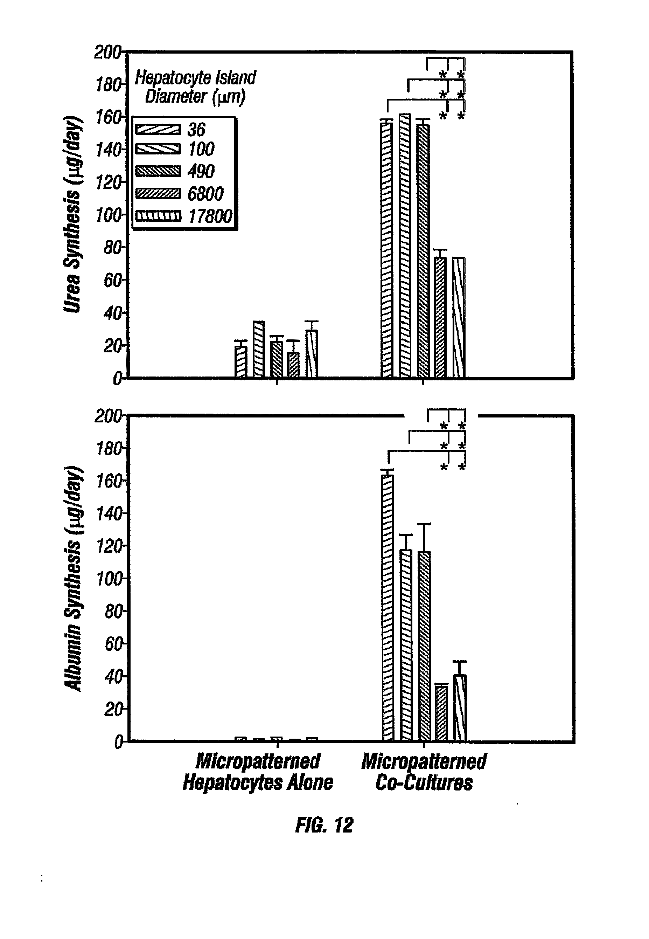

Figure 12 depicts liver-specific function of micropatterned rat cocultures with constant ratio of cell populations. Albumin and urea secretion varied with heterotypic interactions and was higher for cocultures than for hepatocyte only conditions.

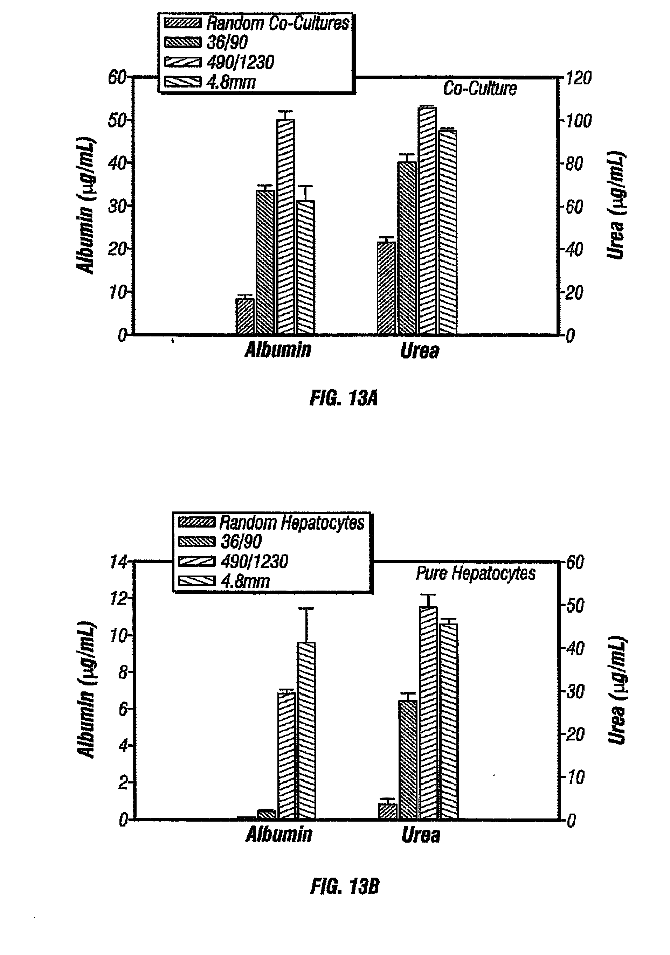

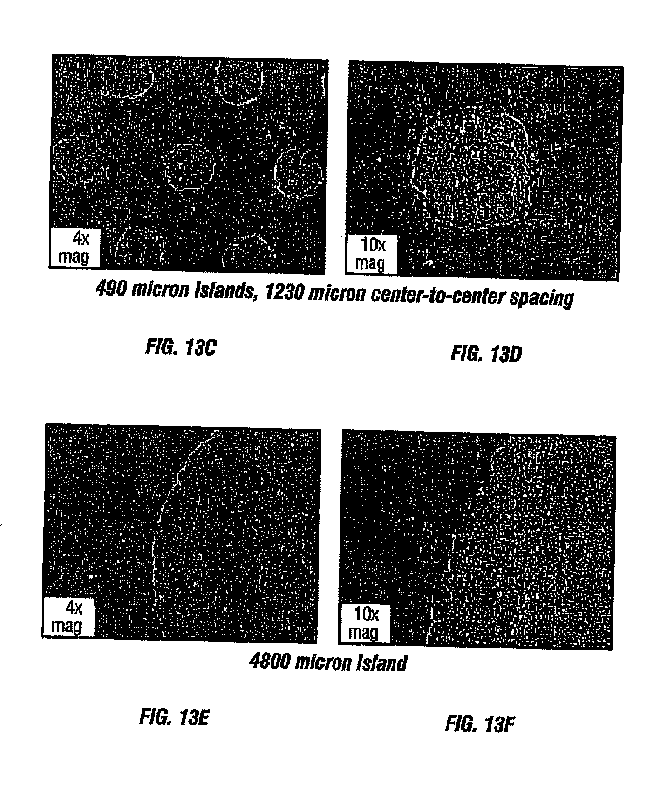

Figure 13 shows optimization of liver-specific functions in human hepatocyte cultures/co-cultures via micro-patterning. Micropatterned cultures/cocultures performed better than randomly seeded ones. 'Random' indicates Randomly seeded cultures, '36/90' indicates 36µm islands separated by 90µm center-to-center spacing, 490/1230 indicates 490µm islands separated by 1230µm center-to-center spacing, and 4.8mm indicates 7 x 4.8mm islands packed in a hexagonal array. These dimensions were chosen to keep the ratio of two cell types and total cell numbers constant. Graphs show hepatocyte function for a representative day 7, while trends were observed for several days. Micrographs of micropatterned cocultures are shown in which hepatocyte islands are surrounded by 3T3-J2 fibroblasts.

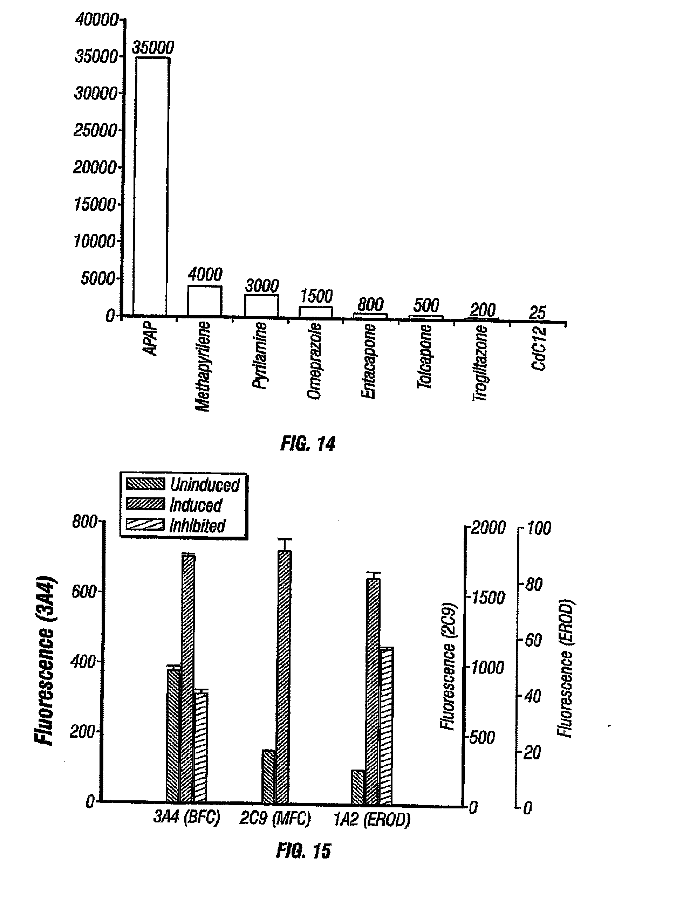

Figure 14 is a graph depicting acute toxicity assays on optimized micropatterned human cocultures, LD₅₀ refers to the dose of the drug (on abscissa) at which the viability signal decreased to 50% of the drug-free control.

Figure 15 is a graph depicting the induction and inhibition of specific CYP450 enzymes. Commercially available fluorescent molecules are used as readouts for these assays.

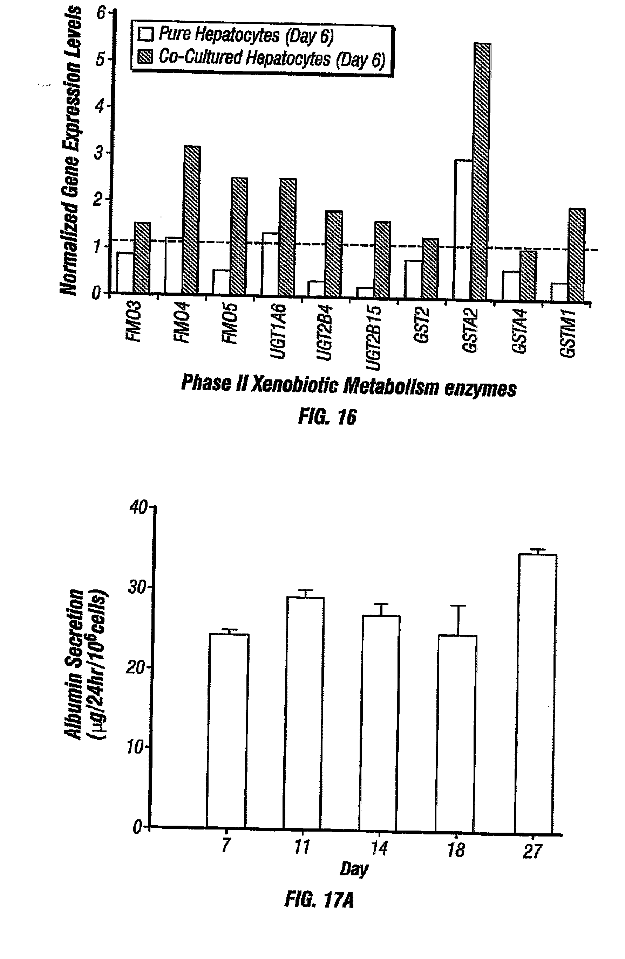

Figure 16 shows the expression profiles of important liver-specific genes in cocultured hepatocytes as compared to pure hepatocytes. RNA was isolated from day 6 old cocultured hepatocytes and pure hepatocyte monolayers and hybridized to Affymetrix Human GeneChip arrays that contain probes for close to 40,000 transcripts. Many important liver-specific genes that are involved in drug metabolism pathways were selected as indicators of the stability of micropatterned human cocultures. Gene expression levels for graphs A-C are normalized to pure hepatocyte gene expression levels on day 1.

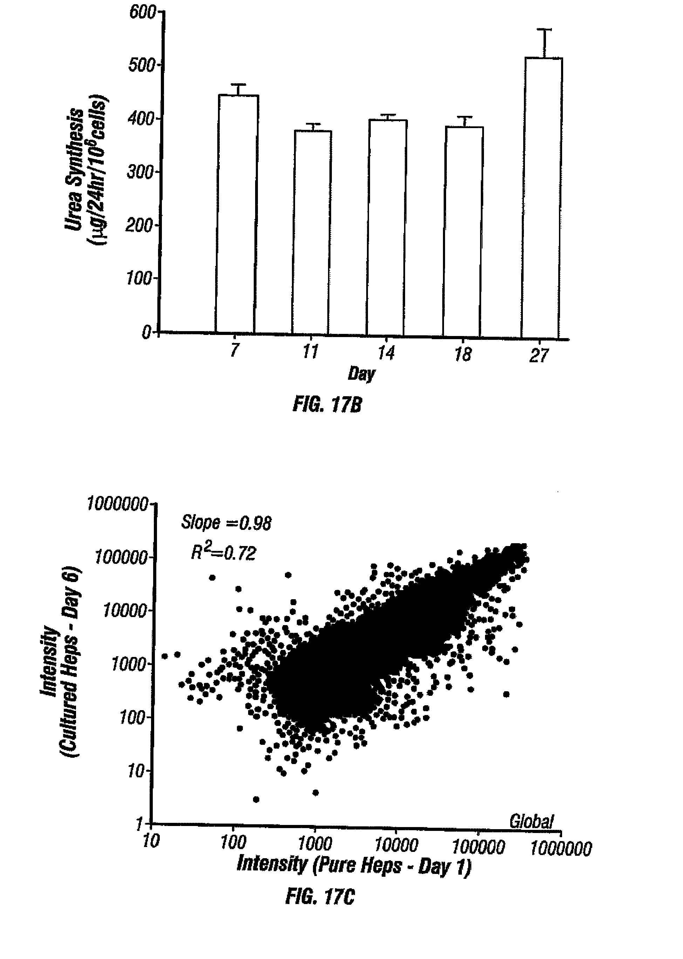

Figure 17A-H shows characterization of microscale human liver tissues. A-B) Rate of albumin secretion and urea production over several weeks in micropatterned co-cultures. C) Global scatter plot comparing gene expression intensities (acquired via Affymetrix GeneChips) in human hepatocytes purified from microscale human liver tissues (day 6) to expression intensities in fresh hepatocytes (12 hours of adherent culture, day 1). D) Scatter plot limited to phase II xenobiotic metabolism genes (*i.e*. UDB-glycosyltransferases glutathione tranaferase). E) Quantitative comparison of cytochrome-P450 (phase I) mRNA in hepatocytes from microscale human liver tissues to pure hepatocyte monolayers, both after one week of culture. All data normalized to gene expression levels in pure hepatocyte monolayers on day 1. F) Quantitative comparison as in 'e' of a panel of key liver-specific genes: ALB, albumin; TF, transferrin (secreted protein); ARG I, arginase I (urea cycle enzyme); G6P, glucose-6-phoaphatase (gluconeogenesis enzymes); F1, BP, fructose 1,6-bisphosphatase (gluconeogenesis enzyme); MDR1, multi-drug resistance gene (pglycoprotein, drug transporter); MRP 1, Multi-drug resistance protein (drug transporter); PXR, pregnane X receptor (nuclear receptor, regulator of xenobiotic metabolism); Factor II and VII are coagulation factors; AsGPR-2, Asialoglycoprotein receptor 2. G) Activity of phase I CYP450 enzymes measured by coumarin analogs in microscale human liver tissues at baseline (untreated, 1 week) and upon treatment with competitive inhibitors. Specific activities of CYP 3A4, 2C9 and 2A6 were demonstrated using substrate/inhibitor combinations: BFC/ketoconazole, MFC/sulfaphenazole and Coumarin/methoxsalen, respectively (MFC, 7-methoxy-4-trifluoromethylcoumarin; BFC, 7-benzyloxy-4-trifluoromethylcoumarin). H) Activity of phase II enzymes monitored by conjugation of glucuronic acid and sulfate groups to 7-Hydroxycoumarin (7-HC) in microscale Human liver tissues (day 10). Amount of 7-HC conjugation, was determined by incubating supernatants from treated cells with B-glucuronidase/arylsulfatase and salicylamide was used as a competitive inhibitor. All error bars represent standard error of the mean (n = 3).

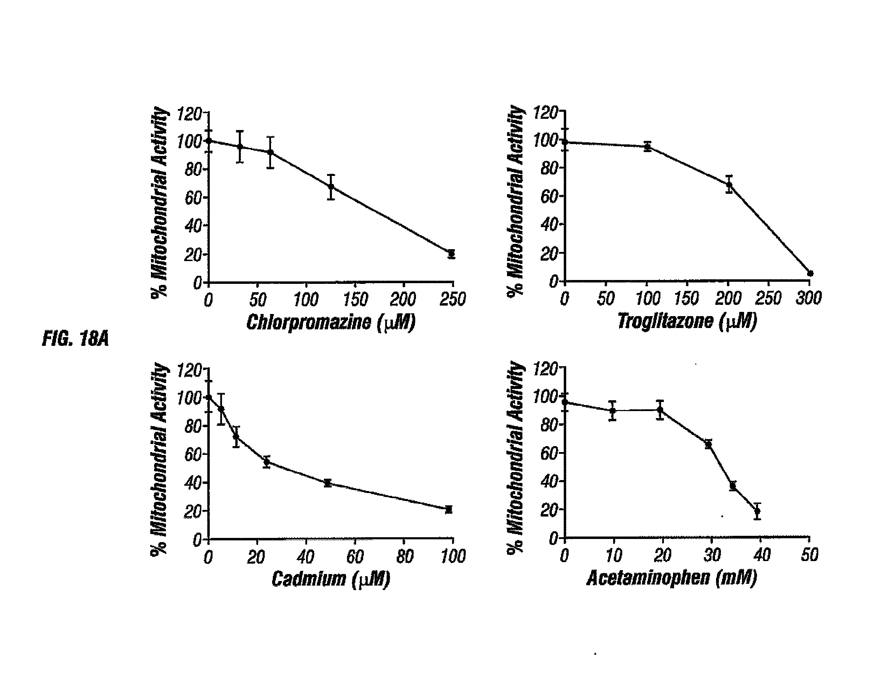

Figure 18A-C shows case studies demonstrating utility of microscale human liver tissues in drug development. A) Dose-dependent toxicity profiles of model hepatotoxins after acute exposure (24 hours). Mitochondrial activity was measured using the MTT assay. All data was normalized to vehicle-only controls. B) Dose and time dependent induction in CYP1A activity upon incubation of microscale tissues for 1 or 3 days with a prototypic inducers, B-Naphthoflavone. ER, Ethoxy-resorufin. C) Dose dependent inhibition of CYP2A6 activity upon incubation of microscale tissues with a prototypic inhibitor, Methoxsalen. An inhibitor of CYP2C9 activity, Sulfaphenazole, showed no inhibition of coumarin 7-hydroxylation even when a 'high' dose (25µM) was utilised. Error bars represent standard error of the mean (n = 3).

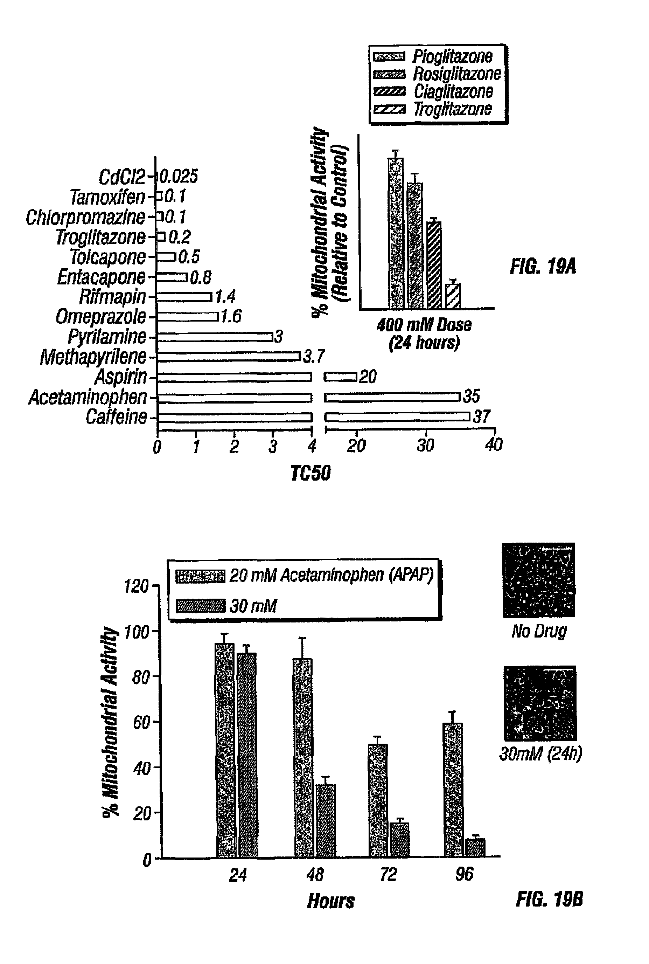

Figure 19A-D shows the utility of microscale human liver tissues in drug development. A) Rank ordering of a panel of compounds including several known hepatotoxins by TC50 - defined as the toxic concentration of drug which produces 50% decrease in mitochondrial activity after 24 hours of exposure to 1-week old tissues (acute toxicity). Mitochondrial toxicity was evaluated using the MTT assay. Inset classifies relative toxicity of structurally-related PPAR-gamma agonists (24 hour exposure at 400µM). All data were normalized to a vehicle only control. B) Time and dose-dependent chronic toxicity of acetaminophen (APAP) in microscale human liver tissues (1-week old). Tissues were dosed repeatedly every 48 hours. All data was normalized to mitochondrial activity in untreated cultures (100% activity). Phase micrographs show human hepatocyte morphology under untreated conditions and after treatment with 30mM of APAP for 24 hours (scale bars is 100µm). C) Induction of CYP450 enzyme activity in microscale human liver tissues using prototypic clinical inducers. Cultures were treated for 4 days before incubation with fluorimetric CYP450 substrates. All data was normalized to vehicle-only controls (fold change of 1). MFC, 7-methoxy-4-trifluoromethylcoumarin; BFC, 7-benzyloxy-4-trifluoromethylcoumarin; COU, Coumarin; ER, Ethoxy-resorufin. d. Species-specific induction of CYP1A isoforms in rat and human microscale tissues using prototypic inducers, β-Naphthoflavone (β-NF) and Omeprazole (OME). Tissues were induced for 4 days and CYP1A activity was assessed via the dealkylation of ethoxyresorufin. Data were normalized to vehicle-only controls. All error bars represent standard error of the mean (n = 3).

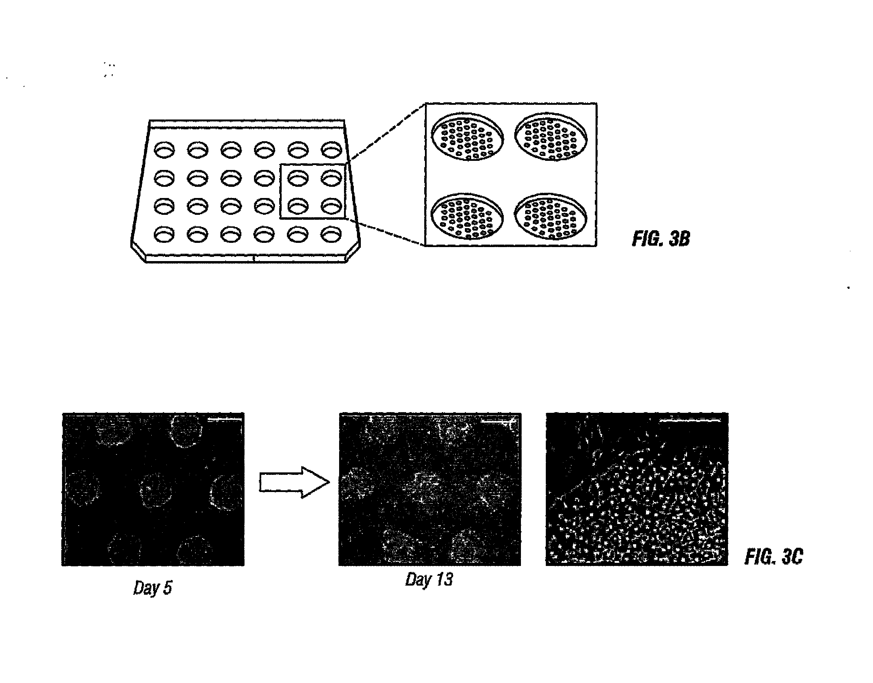

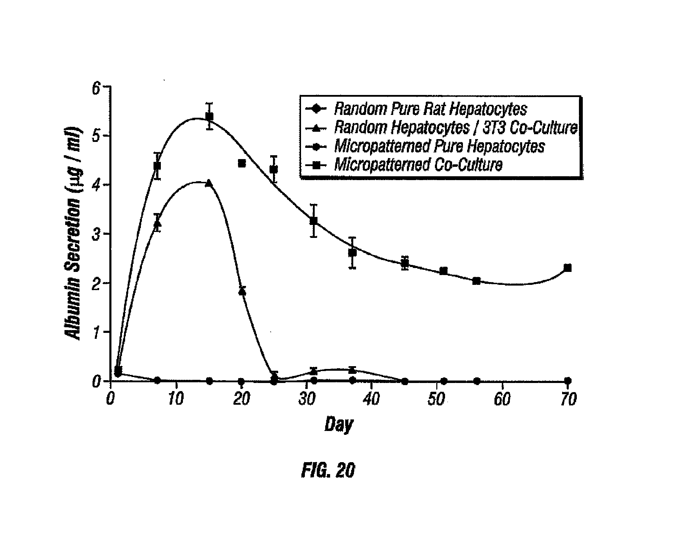

Figure 20 shows microscale engineered model of the rat liver. Primary rat hepatocytes were organized into 500µm islands (1230µm center-to-center spacing) and then surrounded by growth-arrested 3T3-J2 fibroblasts using the soft-lithographic process outlined in Figure 3 ('Micropatterned). Randomly distributed co-cultures ('Random') with similar cell numbers and ratios were generated to enable comparisons. Shown here is rate of albumin production in pure hepatocyte cultures and co-cultures (random and micropatterned) over 70 days. Error bars represent standard error of the mean (n = 3).

Like reference symbols in the various drawings indicate like elements.

### DETAILED DESCRIPTION

As used herein and in the appended claims, the singular forms "a," "and," and "the" include plural referents unless the context clearly dictates otherwise. Thus, for example, reference to "a cellular island" includes a plurality of such cellular islands and reference to "the cell" includes reference to one or more cells known to those skilled in the art, and so forth.

Unless defined otherwise, all technical and scientific terms used herein have the same meaning as commonly understood to one of ordinary skill in the art to which this disclosure belongs. Although methods and materials similar or equivalent to those described herein can be used in the practice of the disclosed methods and compositions, the exemplary methods, devices and materials are described herein.

The publications discussed above and throughout the text are provided solely for their disclosure prior to the filing date of the present applications. Nothing herein is to be construed as an admission that the inventors are not entitled to antedate such disclosure by virtue of prior disclosure.

The invention extends parenchymal cell-stromal cell cocultures by utilizing defined bounded geometries defining cell types. In one aspect, the invention extends cocultures such as those previously used for rat and porcine liver models, to a model of human tissue (*e.g.*, human liver). Using microfabrication tools, the invention demonstrates that micropatterned configurations (from single cellular islands to large aggregates) outperform randomly distributed cocultures. Amongst the micropatterned configurations that were engineered, a balance of homotypic and heterotypic interactions can yield functional cocultures having defined or desired phenotypic activity, longevity and proliferative capacity. Such unexpected results demonstrate a different architectural dependence on geometric cocultures as compared with random cocultures. The invention provides characterization of such micropatterned cocultures utilizing antibody-based functional assays as well as DNA microarrays.

The morphology and function of cells in an organism vary with respect to their environment, including distance from sources of metabolites and oxygen as well as homotypic and heterotypic cell interactions. For example, the morphology and function of hepatocytes are known to vary with position along the liver sinusoids from the portal triad to the central vein (Bhatia et al., Cellular Engineering 1:125-135, 1996; Gebhardt R. Pharmaol Ther. 53(3):275-354, 1992; Jungermann K. Diabete Metab. 18(1):81-86, 1992; and Lindros, K.O. Gen Pharmacol. 28(2):191-6, 1997). This phenomenon, referred to a zonation, has been described in virtually all areas of liver function. Oxidative energy metabolism, carbohydrate metabolism, lipid metabolism, nitrogen metabolism, bile conjugation, and xenobiotic metabolism, have all been localized to separate zones. Such compartmentalization of gene expression is thought to underlie the liver's ability to operate as a 'glucostat' as well as the pattern of zonal hepatotoxicity observed with some xenobiotics *(e.g.,* environmental toxins, chemical/biological warfare agents, natural compounds such as holistic therapies and nutraceuticals).

Isolated human parenchymal cells (such as hepatocytes) are highly unstable in culture and are therefore of limited utility for studies on drug toxicity, drug-drug interaction, drug-related induction of detoxification enzymes, and other phenomena. In spite of their recognized advantages, primacy parenchymal cells are notoriously difficult to maintain in culture as they rapidly lose viability and phenotypic functions upon isolation from their *in vivo* microenvironment. Isolated hepatocytes rapidly lose important liver-specific functions such as albumin secretion, urea synthesis and cytochrome P450 activity (see, *e.g.*, Figure 1). After about a week in culture on collagen-coated dishes, hepatocytes show a fibroblastic morphology. Freshly isolated hepatocytes, on the other hand, show a polygonal morphology with distinct nuclei and nucleoli and bright intercellular boundaries (bile canaliculi). De-differentiated hepatocytes are typically unresponsive to enzyme inducers, which severely limits their use.

Over the last couple of decades, investigators have been able to stabilize several hepatocyte functions using soluble factor supplementation, extracellular matrix manipulation, and random co-culture with various liver and non-liver derived stromal cell types, Addition of low concentrations of hormones, corticosteroids, cytokines, vitamins, or amino acids can help stabilize liver-specific functions in hepatocytes. Presentation of extracellular matrices of different composition and topologies can also induce similar stabilization. For instance, hepatocytes from a variety of species (human, mouse, rat) secrete albumin when sandwiched between two layers of rat tail collage-1 (double-gel). However, studies have shown that CYP450 activities decline in the double-gel model, and the presence of an overlaid layer of collagen presents transport barriers for drug candidates, thus limiting their use as assay systems. Culture on a tumor-derived basement membrane extract called Matrigel also induces hepatocyte spheroid formation and leads to retention of key hepatocyte functions including P450 activity. While Matrigel can induce functions in rodent hepatocytes, it appears to have fewer effects on human hepatocyte. Though they may find use in specific scenarios during drug discovery and development, most *in vitro* liver models in use have limited applicability to the development of a robust biomimetic liver platform. For instance, defined media formulations limit the contents of the perfusate, sandwich culture adds a transport barrier and Hepatocytes do not express gap junctions, and Matrigel and spheroid culture rely on hepatocyte aggregation with resultant non-uniformity and transport barriers.

The Invention overcomes many of these problems by optimizing the homotypic and heterotypic interactions of parenchymal cells with non-parenchymal cells. For example, in the adult liver, hepatocytes interact with a variety of stromal cell types including sinusoidal endothelia, stellate cells, Kupffer cells and fat-storing Ito cells (*e*.*g*., heterotypic interactions). These stromal cell types modulate cell fate processes of hepatocytes, under both physiologic and pathophysiologic conditions. *In vitro*, random co-cultivation of primary hepatocytes with a plethora of distinct stromal cell types from different species and organs has been shown to support differentiated hepatocyte function for Several weeks in a manner reminiscent of hepatic organogenesis (see Figure 2). These random hepatocyte co-cultures have been used to study various aspects of liver physiology and pathophysiology such as lipid metabolism, and induction of the acute-phase response.

In one aspect, micropatterned cultures comprising cellular islands of parenchymal cells and stromal cells are used. In this aspect, a substrate is modified and prepared such that stromal cells are interspersed with islands of parenchymal cells. Using microfabrication techniques modified, for example, from the semiconductor industry, the substrate is modified to provide for spatially arranging parenchymal cells *(e.g.,* human hepatocytes) and supportive stromal cells (*e. g.,* fibroblasts) in a miniaturizable format. The spatial arrangements can be a parenchymal cell type comprising a bounded geometric shape. The bounded geometric shape can be any shape *(e.g.,* regular or irregular) having dimensions defined by the shape (*e*.*g*., diameter, width, length and the like). The dimensions will have a defined scale based upon their shape such that at least one distance from one side to a substantially opposite side is about 200-800 µm (*e*.*g*., where the shape is rectangular or oval, the distance between one side to an opposite side is 200-800 µm). For example, parenchymal cells (*e*.*g*., hepatocytes) can be prepared in circular islands of varying dimensions (*e.g*., 36µm, 100µm, 490µm, 4.8mm, and 12.6mm in diameter; typically about 250-750 µm) surrounded by stromal cells (*e.g*., fibroblast such as murine 3T3 fibroblasts) or other materials. For example, hepatocyte detoxification functions are maximized at small patterns, synthetic ability at intermediate dimensions, while metabolic function and normal morphology were retained in all patterns.

In one embodiment, a bioreactor can use primary parenchymal cells (*e*.*g.,* hepatocytes) alone or in combination with other cell types. Although the examples provided herein utilize hepatocytes, other parenchymal and non-parenchymal cell types that can be used in the bioreactors and cultures systems of the disclosure include pancreatic cells (alpha, beta, gamma, delta), myocytes, enterocytes, renal epithelial cells and other kidney cells, brain cell (neurons, astrocytes, glia), respiratory epithelium, stem cells, and blood cells (*e.g*., erythrocytes and lymphocytes), adult and embryonic stem cells, blood-brain barrier cells, and other parenchymal cell types known in the art.

In one aspect, the reactor can be used with micropatterned parenchymal (*e*.*g*., hepatocytes) co-cultures and stromal cells (*e.g*., fibroblasts). The scale of the reactor can be altered to allow for the fabrication of a high-throughput microreactor array to allow for interrogation of xenobiotics. In one aspect, a microfluidic device is contemplated that has micropatterned culture areas in or along a fluid flow path.

The invention demonstrates that cell-cell interactions, both homotypic (hepatocyte/hepatocyte) and heterotypic (hepatocyte/stromal), improve viability and differentiated function of parenchymal cells.

The micropatterned cell island cultures of the invention are useful in drug discovery and development including screening for metabolic stability, drug-drug interactions, toxicity and infectious disease. Metabolic stability is a key criterion for selection of lead drug candidates that proceed to preclinical trials.

The invention provides a cellular composition useful for the development of in vitro tissues with desired characteristics and/or the ability to be cultured over long periods of time with minimal de-differentiation. The invention is based, in-part, upon the discovery that distances between homotypic cell populations and their relationship to intervening heterotypic cell populations results in various functional (phenotypic) differences. For example, in one aspect of the invention one or more populations of geometrically defined cellular islands comprising parenchymal cells are generated. As described further herein, the parenchymal cell type may be any parenchymal cell. The specific examples, provided below demonstrate the application of the methods and systems to hepatic parenchymal cells. These parenchymal cell islands are surrounded/separated by a population of non-parenchymal cells.

The cellular islands can take any geometric shape having a desired characteristic and can be defined by length/width, diameter and the like, based upon their geometric shape, which may be circular, oval, square, rectangular, triangular and the like. Furthermore parenchymal cell function may be modified by altering the pattern configuration (*e*.*g*., the distance or geometry of the array of cellular islands). The distance between bounded geometric islands of cells may vary in a culture system (*e*.*g*., the distances between islands may be regular or irregular). Using techniques described herein, the spatial distances between cellular islands may be random, regular or irregular. Furthermore, combinations of geometric bounded areas (*e*.*g*., cellular islands) of different geometries (*e*.*g*., multiple island sizes) may be present on a single substrate with varying distances (*e*.*g*., multiple island spacings) or regular distances between the islands. In other words, the invention contemplates the use of cellular islands comprising various geometries and distances on a substrate (*e*.*g*., cocultures comprising cellular islands with 250 µm and 400 µm islands that are intermixed and regularly distributed). In one aspect, the Cellular islands comprise a diameter or width from about 250µm to 750µm. Similarly, where the geometric island comprises a rectangle the width can comprise about 250µm to 750µm. In another aspect, the parenchymal cellular islands are spaced apart from one another by about 2µm to 1300 µm from center to center of the cellular islands. In yet a further aspect, the parenchymal cell islands comprise a defined width (*e.g*., 250µm to 750µm) that can run the length of a culture area or a portion of the culture area. Parallel islands of parenchymal cells can be separated by parallel rows of stromal cells. In another aspect, the geometric shape may comprise a 3-D shape (*e*.*g*., a spheroid). In such instances, the diameter/width and the like, will be from about 250µm to 750µm.

As will be recognized in the art, the cellular islands may be present in any culture system including static and fluid flow reactor systems (*e*.*g*., microfluidic devices). Such microfluidic devices are useful in the rapid screening of agents where small flow rates and small reagent amounts are required.

The cellular culture of the Invention can be made by any number of techniques that will be recognized in the art. For example, a method of making a plurality of cellular islands on a substrate can comprise spotting or layering an adherence material (or plurality of different cell specific adherence materials) on a substrate at spatially different locations each spot having a defined size (*e*.*g*., diameter) and spatial arrangement. The spots on the substrate are then contacted with a first cell population or a combination of cell types and cultured to generate cellular islands. Where difference cell-types are simultaneously contacted with the substrate, the substrate, coating or spots on the substrate will support cell-specific binding, thus providing distinct cellular domains. Methods for spotting adherence material (*e*.*g*., extracellular matrix material) can include, for example, robotic spotting techniques and lithographic techniques.

Various culture substrates can be used in the methods and systems of the invention. Such substrates include, but are not limited to, glass, polystyrene, polypropylene, stainless steel, silicon and the like. The choice of the substrate should be taken into account where spatially separated cellular islands are to be maintained. The cell culture surface can be chosen from any number of rigid or elastic supports . For example, cell culture material can comprise glass or polymer microscope slides. In some aspect, the substrate may be selected based upon a cell type's propensity to bind to the substrate.

The cell culture surface/substrate used in the methods and systems of the invention can be made of any material suitable for culturing mammalian cells. For example, the substrate can be a material that can be easily sterilized such as plastic or other artificial polymer material, so long as the material is biocompatible. A substrate can be any material that allows cells and/or tissue to adhere (or can be modified to allow cells and/or tissue to adhere or not adhere at select locations) and that allows cells and/or tissue to grow in one or more layers. Any number of materials can be used to form the substrate/surface, including, but not limited to, polyamides; polyesters; polystyrene; polypropylene; polyacrylates; polyvinyl compounds (*e.g.* polyvinylchloride); polycarbonate (PVC); polytetrafluoroethylene (PTFE); nitrocellulose; cotton; polyglycolic acid (PGA); cellulose; dextran; gelatin, glass, fluoropolymers, fluorinated ethylene propylene, polyvinylidene, polydimethylsiloxane, polystyrene, and silicon substrates (such as fused silica, polysilicon, or single silicon crystals), and the like. Also metals (gold, silver, titanium films) can be used.

As mentioned herein, in some instances the substrate may be modified to promote cellular adhesion and growth (*e*.*g*., coated with an adherence material) . For example, a glass substrate may be treated with a protein (*i*.*e*., a peptide, of at least two amino acids) such as collagen or fibronectin to assist cells in adhering to the substrate. In some embodiments, the proteinaceous material is used to define the location of a cellular island. The spot produced by the protein serves as a "template" for formation of the cellular island. Typically, a single protein will be adhered to the substrate, although two or more proteins may be used in certain embodiments. Proteins that are suitable for use in modifying a substrate to facilitate cell adhesion include proteins to which specific cell types adhere under cell culture conditions. For example, hepatocytes are known to bind to collagen. Therefore, collagen is well suited to facilitate binding of hepatocytes. Other suitable proteins include fibronectin, gelatin, collagen type IV, laminin, entactin, and other basement proteins including glycosaminoglycans such as heparin sulfate. Combinations of such proteins also can be used.

The type of adherence material(s) (*e*.*g*., ECM materials, sugars, proteoglycans *etc*.) deposited in a spot will be determined in part, by the cell type or types to be cultured. For example, ECM molecules found in the hepatic microenvironment are useful in culturing hepatocytes, the use of primary cells, and a fetal liver-specific reporter ES cell line. The liver has heterogeneous staining for collagen I, collagen III, collagen IV, laminin, and fibronectin. Hepatocytes display integrins β1, β2, α1, α2, α5, and the nonintegrin fibronectin receptor Agp110 in vivo. Cultured rat hepatocytes display integrins α1, α3, α5, β1, and α6β1, and their expression is modulated by the culture conditions.

In one aspect, the invention provides a micropatterned hepatocyte co-culture. Due to species-specific differences in drug metabolism, human hepatocyte cultures can identify the metabolite profiles of drug candidates more effectively than non-human cultures. Although, it will be recognized that non-human cell types may be used in the invention to facilitate identification of properties or metabolisms suitable for further study of human cells. This information can then be used to deduce the mechanism by which the metabolites are generated, with the ultimate goal of focusing clinical studies. Though there are quantitative differences, there is good *in* vivo to *in vitro* correlation in drug biotransformation activity when isolated hepatocytes are used. Metabolite profiles obtained via human hepatocyte *in vitro* models can also be used to choose the appropriate animal species to act as the human surrogate for preclinical pharmacokinetic, pharmacodynamic and toxicological studies. Studies have shown that interspecies variations are retained in vitro and are different depending on the drug being tested.

The invention also provides methods of micropatterning useful to develop tissues with desired characteristics. Although a serial photolithographic based technique was used in the specific examples below to create optimized micropatterned cocultures, the studies indicate that such cocultures can be miniaturized using stencil-based soft lithography in a multi-well format amenable for higher throughput experimentation.
Patterning of various combinations and types of extracellular matrix proteins on a single substrate using robotic spotting techniques is also provided by the invention. These matrix arrays coupled with parenchymal *(e.g.,* hepatic) and stromal cocultures are amenable to high-throughput screening in drug development applications. The invention also provides functionally stable 2-D and 3-D cocultures in static and bioreactor settings with closed-loop flow conditions that approximate *in vivo* conditions. Furthermore, the micropatterning strategy can potentially be used to functionally optimize other systems in which cell-cell interactions are important (*e.g*., hematopoietic stem cells co-cultivated with stromal cell lines and keratinocytes with fibroblasts).

With regard to placing insoluble and/or soluble factors at specific locations, various micro-spotting techniques using computer-controlled plotters or even ink-jet printers have been developed to spot such factors at defined locations. One technique loads glass fibers having multiple capillaries drilled through them with different materials loaded into each capillary tube. A substrate, such as a glass microscope slide, is then stamped out much like a rubber stamp on each glass slide. Spotting techniques involve the placement of materials at specific sites or regions using manual or automated techniques.

Conventional physical spotting techniques such as quills, pins, or micropipettors are able to deposit material on substrates in the range of 10 to 250 microns in diameter (*e*.*g.,* about 100 spots/microwell of a 96 well culture plate). In some instances the density can be from 400 to 10000 spots per square centimeter, allowing for clearance between spots. Lithographic techniques, such as those provided by Affymetrix (*e*.*g*., U.S. Pat. No. 5,744,305, the disclosure of which is incorporated by reference herein) can produce spots down to about 10 microns square, resulting in approximately 800,000 spots per square centimeter.

A spotting device may employ one or more piezoelectric pumps, acoustic dispersion, liquid printers, micropiezo dispensers, or the like to deliver such reagents to a desired location on a substrate. In some embodiments, the spotting device comprises an apparatus and method like or similar to that described in U.S. Pat. Nos. 6,296,702, 6,440,217, 6,579,367, and 6,849,127.

Accordingly, an automated spotting device can be utilized, e.g. Perkin Elmer BioChip Arrayer*. A number of contact and non-contact microarray printers are available and may be used to dispense/print the soluble and/or insoluble materials on a substrates. For example, non-contact printers are available from Perkin Elmer (BioChip Arrayer™), Labcyte and IMTEK (TopSpot™), and Bioforce (Nanoarrayer™). These devices utilize various approaches to non-contact spotting, including piezo electric dispension; touchless acoustic transfer; en bloc printing from multiple microchannels; and the like. Other approaches include ink jet-based printing and microfluidic platforms. Contact printers are commercially available from TeleChem International (Arraylt™).

Non-contact printing will typically be used for the production of cellular microarrays comprising cellular islands. By utilizing a printer that does not physically contact the surface of substrate, no aberrations or deformities are introduced, onto the substrate surface, thereby preventing uneven or aberrant cellular capture at the site of the spotted material.

Printing methods of interest, including those utilizing acoustic or other touchless transfer, also provide benefits of avoiding clogging of the printer aperature, *e.g.* where solutions have high viscosity, concentration and/or tackiness. Touchless transfer printing also relieves the deadspace inherent to many systems. The use of print heads with multiple ports and the capacity for flexible adjustment of spot size can be used for high-throughput preparation.

The total number of spots on the substrate will vary depending on the substrate size, the size of a desired cellular island, and the spacing between cellular islands. Generally, the pattern present on the surface of the support will comprise at least 2 distinct spots, usually about 10 distinct spots, and more usually about 100 distinct spots, where the number of spots can be as high as 50,000 or higher. Typically, the spot will usually have an overall circular dimension (although other geometries such as spheroids rectangles, squares and the like may be used) and the diameter will range from about 10 to 5000 µm (*e*.*g*., about 200 to 800 µm).

By dispensing or printing onto the surfaces of multi-well culture plates, one can combine the advantages of the array approach with those of the multi-well approach. Typically, the separation between tips in standard spotting device is compatible with both a 384 well and 96 well plates; one can simultaneously print each load in several wells. Printing into wells can be done using both contact and non-contact technology.

The invention can utilize robotic spotting technology to develop a robust, accessible method for forming cellular microarrays or islands of a defined size and spatial configuration on, for example, a cell culture substrate. As used herein, the term "microarray" refers to a plurality of addressed or addressable locations.

In one aspect, the invention provides methods and systems comprising a modified printing buffer used in a spotting device to allow for ECM deposition, and identifying microarray substrates that permit ECM immobilization. The methods and systems of the invention are useful for spotting substantially purified or mixtures of biological proteins, nucleic acids and the like (*e*.*g*., collagen I, collagen III, collagen IV, laminin, and fibronectin) in various combinations on a standard cell culture substrate (*e*.*g*., a microscope slide) using off-the-shelf chemicals and a conventional DNA robotic spotter.

In another aspect, the invention utilizes photolithographic techniques to generate cellular islands. Drawing on photolithographic micropatterning techniques to manipulate functions of rodent hepatocytes upon co-cultivation with stromal cells, a microtechnology-based process utilizing elastomeric stencils to miniaturize and characterize human liver tissue in an industry-standard multiwell format was used. The approach incorporates 'soft lithography,' a set of techniques utilizing reusable, elastomeric, polymer (Polydimethylsiloxane-PDMS) molds of microfabricated structures to overcome limitations of photolithography. In one aspect, the invention provides a process using PDMS stencils consisting of 300µm thick membranes with through-holes at the bottom of each well in a 24-well mold (Fig. 3a). To micropattern all wells simultaneously, the assembly was sealed against a polystyrene plate . Collagen-I was physisorbed to exposed polystyrene, the stencil was removed and a 24-well PDMS 'blank' was applied. Co-cultures were 'micropatterned' by selective adhesion of human hepatocytes to collagenous domains, which were then surrounded by supportive murine 3T3-J2 fibroblasts. The size (*e*.*g*., geometric dimension) of through-holes determined the size of collagenous domains and thereby the balance of homotypic (hepatocyte/hepatocyte) and heterotypic (hepatocyte/stroma) interactions in the microscale tissue. Similar techniques can be used to culture cellular islands of other parenchymal cell types.

The Invention provides methods and systems useful for identifying optimal conditions for controlling cellular development and maturation by varying the size and/or spacing of a cellular island. For example, the methods and systems of the invention are useful for identifying optimal conditions that control the fate of cells (*e.g*., differentiating stem cells into more mature cells, maintenance of self-renewal, and the like).

The term "adherence material" is a material deposited on a substrate or chip to which a cell or microorganism has some affinity, such as a binding agent. The material can be deposited in a domain or "spot". The material, and a cell or microorganism interact through any means including, for example, electrostatic or hydrophobic interactions, covalent binding or ionic attachment. The material may include, but is not limited to, antibodies, proteins, peptides, nucleic acids, peptide aptamers, nucleic acid aptamers, sugars, proteoglycans, or cellular receptors.

In a specific example, the Invention provides methods and compositions useful to optimize hepatocyte function *in vitro.* The invention extends hepatocyte-fibroblast cocultures, previously used for rat and porcine liver models, to a model of human liver. Using microfabrication tools, the invention demonstrates that micropatterned configurations (from single hepatocyte islands to large aggregates) outperform randomly distributed cocultures. Amongst the micropatterned configurations that were engineered, a clear balance of homotypic and heterotypic interactions can yield functional human cocultures. Such unexpected results demonstrate a different architectural dependence in human cocultures as compared with rat cocultures. The characterization of optimized micropatterned human cocultures is extensive, utilizing antibody-based functional assays as well as DNA microarrays. Studies in cocultured liver tissue indicate that micropatterned human cocultures retain a high level of expression of many important liver-specific genes, while a decline in expression is seen in pure hepatocyte monolayers on collagen, which are commonly used during drug development. The validation of human cocultures as appropriate liver models for drug development includes cell-based acute and chronic toxicity assays using a variety of clinical and non-clinical compounds, as well as induction and inhibition of key CYP450 enzymes.

A cellular island or "spot" refers to a bounded geometrically defined shape of a substantially homogenous cell-type having a defined border. In one aspect, the cellular island or spot is surrounded by different cell-types, materials (*e*.*g*., extracellular matrix materials) and the like. The cellular islands can range in size and shape (*e.g*., may be of uniform dimensions or non-uniform dimensions). Cellular islands may be of different shapes on the same substrate. Furthermore, the distance between two or more cellular islands can be designed using methods known in the art *(e.g.,* lithographic methods and spotting techniques). The distances between cellular islands can be random, regular or irregular. The distance between and/or size of the cellular islands can be modified to provide a desired phenotypic characteristic of morphology to a particular cell types *(e.g.,* a parenchymal cell such as a hepatocyte).

In addition to modulating cellular islands to control heterotypic and/or homotypic interactions the invention can use a bioreactor system that provides the ability to modulate oxygen and nutrient uptake processes of mammalian cells to create a directional gradient in a reactor system. Directional oxygen gradients are present in various biological environments such as, for example, in cancer, tissue development, tissue regeneration, wound healing and in normal tissues. As a result of oxygen gradients along the length of a bioreactor system result in cells exhibiting different functional characteristics based on local oxygen availability. Accordingly, the invention provides methods, reactor systems and compositions that provide the ability of develop human tissues *in vitro* characteristic of normal tissue, but also to provide similar physiological environments by mimicking oxygen and/or nutrient gradients found in tissues in the body.

The use of the micropattern technology in combination with a bioreactor system allows for the development of microarray bioreactors. Previous bioreactors were not amenable to miniaturization due in part to variable tissue organization due to reliance on self-assembly that underlie variations in nutrient and drug transport, and uncharacterized stromal contaminants (*e*.*g*., random Cultures). Furthermore, previous random cho-cultures have uncharacterized stromal cell population, have difficulty with microscopic imaging, have difficulty assessing cell number (due to proliferating cell populations) and display less cell-specific (*e.g.,* liver specific) function than micropatterned co-cultures. The micropatterning provided by the invention overcomes many of these difficulties.

The bioreactor utilizes co-cultures of cells in which at least two types of cells are configured in a bounded geometric pattern on a substrate. Such micropatterning techniques are useful to modulate the extent of heterotypic and homotypic cell-cell contacts. In addition, co-cultures have improved stability and thereby allow chronic testing (*e*.*g*., chronic toxicity testing as required by the Food and Drug Administration for new compounds). Because micropatterned co-cultures are more stable than random cultures the use of co-cultures of the invention and more particularly micropatterned co-cultures provide a beneficial aspect to the cultures systems of the disclosure. Furthermore, because drug-drug interactions often occur over long periods of time the benefit of stable co-cultures allows for analysis of such interactions and toxicology measurements.

In one aspect, the invention provides an in vitro model of human liver tissue that can be utilized for pharmaceutical drug development, basic science research, infectious disease research (*e.g*., hepatits B, C and malaria) and in the development of tissue for transplantation. The invention provides compositions, methods, and bioreactor systems that allow development of long-term human cultures *in vitro*. In addition, the compositions, methods and bioreactor systems of the invention provide for the design of particular morphological characteristics by modifying cellular island size and distribution. The compositions, methods and bioreactor systems of the disclosure have been applied to liver cultures and have shown that cellular island size and/or distribution contribute to induction of cellular metabolism that mimics *in* vivo metabolism. The results demonstrate that cellular distribution modulates gene expression and imply an important role in the maintenance of cell-specific metabolism (*e*.*g*., liver specific metabolism). In addition, considerations of the effect of such distribution in the design and optimization of current bioartificial support systems may serve to improve their function.

Characterization using antibody-based functional assays as well as DNA microarrays has demonstrated long-term liver-specific stability (protein and RNA levels) of micropatterned human cocultures of the invention. To demonstrate applications in drug development, acute acute/chronic toxicity assays as well as induction/inhibition of cytochrome P450s (key drug metabolism enzymes) via classic drugs were conducted. For instance, in vitro work with the drug REZULIN (Troglitazone), which was withdrawn from the market in 2000 due to idiosyncratic (1 in 10,000 occurrences) liver toxicity, show that this drug is considerably more toxic than a commonly used analgesic, acetaminophen (active ingredient in Tylenol). Accordingly, the invention is useful to screen for toxicity and drug interactions the may have either positive or negative effects on cellular metabolism.

The tissue cultures and bioreactors of the disclosure may be used to *in vitro* to screen a wide variety of compounds, such as cytotoxic compounds, growth/regulatory factors, pharmaceutical agents, and the like, to identify agents that modify cell *(e.g.,* hepatocyte) function and/or cause cytotoxicity and death or modify proliferative activity or cell function. For example, the culture system may be used to test adsorption, distribution, metabolism, excretion, and toxicology (ADMET) of various agents. To this end, the cultures are maintained *in vitro* comprising a defined cellular island geometry and exposed to a compound to be tested. The activity of a compound can be measured by its ability to damage or kill cells in culture or by its ability to modify the function of the cells *(e.g.,* in hepatocytes the expression of P450, and the like). This may readily be assessed by vital staining techniques, ELISA assays, immunohistochemistry, and the like. The effect of growth/regulatory factors on the cells *(e.g.,* hepatocytes, endothelial cells, epithelial cells, pancreatic cells, astrocytes, muscle cells, cancer cells) may be assessed by analyzing the cellular content of the culture, *e.g.,* by total cell counts, and differential cell counts or by metabolic markers such as MTT and XTT. This may also be accomplished using standard cytological and/or histological techniques including the use of immunocytochemical techniques employing antibodies that define type-specific cellular antigens. The effect of various drugs on normal cells cultured in the culture system may be assessed. For example, drugs that affect cholesterol metabolism, *e.g.,* by lowering cholesterol production, could be tested on a liver culture system.

The methods and systems of the invention can be used to make and assay models of both normal and abnormal tissue. For example, surrounding hepatocytes with activated stellate cells would mimic fibrotic liver tissue. In this aspect, hepatocytes islands are generated and are bordered by activated stellate cells. Alternatively, pathologic liver tissue may be used as a source of hepatocytes that are used in the formation of cellular islands . These abnormal hepatocytes can be bordered by normal or abnormal non-parenchymal cell types.

In another aspect, infectious diseases may be monitored in the presence and absence of test agents. For example, in liver cultures of the invention hepatitis B and C may be tested for their effects on heterotypic and homotypic interactions as well as interactions on particular cells. Furthermore, test agents used to treat such diseases may be studies. Similarly, malaria and other infectious diseases and potential therapeutics may be tested.

One advantage of the bioreactor and culture systems of the disclosures (*e*.*g*., a single as well as an array of bioreactors of the invention) is that the cells in such a bioreactor or culture system are substantially homogenous and autologous (*e*.*g.,* the cellular islands are substantially homogenous and autologous) so you can do many experiments on the same biological background. *In vivo* testing, for example, suffers from animal-to-animal variability and is limited by the number of conditions or agents that can be tested on a given subject.

The cytotoxicity to cells in culture (*e*.*g*., human hepatocytes) of pharmaceuticals, anti-neoplastic agents, carcinogens, food additives, and other substances may be tested by utilizing the bioreactor culture system of the disclosure.

In one aspect of the assays system, a stable, growing culture is established within the bioreactor system having a desired size (*e.g.,* island size and distance between islands), morphology and may also include a desired oxygen gradient. The cells/tissue in the culture are exposed to varying concentrations of a test agent . After incubation with a test agent, the culture is examined by phase microscopy or by measuring cell specific functions (*e.g.,* hepatocyte cell indicators) such as protein production/metabolism to determine the highest tolerated dosethe concentration of test agent at which the earliest morphological abnormalities appear or are detected. Cytotoxicity testing can be performed using a variety of supravital dyes to assess cell viability in the culture system, using techniques known to those skilled in the art.

Once a testing range is established, varying concentrations of the test agent can be examined for their effect on viability, growth, and/or morphology of the different cell types by means well known to those skilled in the art.

The bioreactor culture system may also be used to aid in the diagnosis and treatment of malignancies and diseases or in toxicogenomic studies. For example, a biopsy of a tissue (such as, for example, a liver biopsy) may be taken from a subject suspected of having a drug sensitivity, malignancy or other disease or disorder. The biopsy cells can then be cultured in the bioreactor system under appropriate conditions where the activity of the cultured cells can be assessed using techniques known in the art. In addition, such biopsy cultures can be used to screen agent that modify the activity in order to identify a therapeutic regimen to treat the subject to identify genes causing drug sensitivity or toxicology, or disease sensitivity. For example, the subject's tissue culture could be used in *vitro* to screen cytotoxic and/or pharmaceutical compounds in order to identify those that are most efficacious; *i.e.* those that kill the malignant or diseased cells, yet spare the normal cells or to identify drugs that do not cause a toxic response due to drug sensitivities (*e.g.,* screening related to personalized medicine). These agents could then be used to therapeutically treat the subject.

Similarly, the beneficial effects of drugs may be assessed using the culture system in vitro; for example, growth factors, hormones, drugs which enhance hepatocyte formation or activity can be tested. In this case, stable micropattern cultures may be exposed to a test agent. After incubation, the micropattern cultures may be examined for viability, growth, morphology, cell typing, and the like as an indication of the efficacy of the test substances. Varying concentrations of the drug may be tested to derive a dose-response curve.

The culture systems of the invention may be used as model systems for the study of physiologic or pathologic conditions. For example, in a specific embodiment, the culture system can be optimized to act in a specific functional manner as described herein by modifying the size or distribution of cellular islands. In another aspect, the oxygen gradient is modified along with the density and or size of a micropattern of cells in the culture system.

A bioreactor useful in the methods of the invention is generally depicted in FIG. 4. The bioreactor 5 of the comprises a pump 90, a gas exchange device 100, a bubble trap 120, a culture device 15 comprising a substrate 20, a tissue binding surface 30 and bottom surface 40, an enclosure/housing 50 having at least one wall 55, inlet port 60 and outlet port 70, sensor 110, and fluid reservoir 80. The bioreactor 5 comprises a pump 90 used to maintain circulation of fluid in the system. Pump 90 can be in fluid communication with a gas exchange device 100 that oxygenates the fluid present in the system to a desired concentration. The pump 90 is also in fluid communication with fluid reservoir 80 used to contain, for example, nutrient media or other media to be contacted with cells in the system. In one aspect, the gas exchange device 100 is in fluid communication with a bubble trap 120 that serves to remove bubbles following gas exchange of the fluid in the gas exchange device 100. Fluid flowing through the system enters inlet port 60 of culture device 15 and passes across substrate 20 to outlet port 70. The inlet port 50 and outlet port 70 may be located on the x-, y-, or z-plane of the enclosure/housing 50.

In the specific embodiment of FIG. 4 the growth surface for cells is shown as being on top surface 30 of substrate 20, additional surfaces may be prepared for cell adherence and growth including any surface of housing/chamber 50 (*i.e.,* any one or more walls of the chamber 50). In FIG. 4, cells are capable of growth on the top surface 30 of substrate 20. As discussed herein, the substrate 20 or one or more surfaces of housing/chamber 50 may be treated or modified to promote cellular adhesion to the substrate or improve cell growth. Optical transparency of the substrate 20 and/or of the housing/chamber 50 is useful as a platform for conventional microscopy (fluorescent and transmitted light). Furthermore, in-line sensor can be incorporated using microtechnology or nanotechnology can be present to measure various metabolic products or by-products indicative of cellular toxicity and/or growth and viability. For example, molecular probes (*e.g.,* probes that provide a measurable signal such as changes in fluorescence, electrical conductivity (including resistance, capacitance) can be included in the bioreactor to monitor various culture parameters. Probes that can indicate a change include various green fluorescent protein molecules linked to various indicators that change conformation upon interacting with a molecule in the cellular milieu or media effluent. Probes that provide electrical changes upon interacting with a molecule in the cellular milieu or media effluent can include substrates that comprise various polymers (*e.g.* polypyrrole, polyaniline and the like, as well as semiconductive substrates). Such substrates change resistance or capacitance upon interacting with a molecule. For example, each reactor (or a plurality of reactors in a microarray, as described herein) can have its own O₂, pH, and metabolite sensor(s). Other sensor types are known in the art. In addition, methods of microfabrication for inclusion of such sensors are also known in the art.

In one aspect, fluid, upon exiting culture device 15 through outlet port 70, contacts a sensor 110 (*e.g.,* an oxygen sensor, metabolite sensor, and the like) that measures an analyte of interest. The data obtained from the sensor 110 can be used to modulate tissue growth and or to obtain data related to the efficacy or toxicity of a particular agent or drug.

In a further embodiment, the bioreactor system 5 may be used in an array of bioreactor systems as depicted in FIG. 5. FIG. 5 is a schematic representation of a plurality of miniature bioreactor systems 5 in fluid communication. Depicted are inlet port 60 and outlet port 70 for each cell culture device 15. Cells 10 in each culture device 15 are grown on substrate 20 or a plurality of substrates 20.

Referring again to FIG. 4, one embodiment of a bioreactor 5 according to the disclosure has a tissue 10, which is seeded on top portion 30 of substrate 20. A cover chamber or housing 50 comprises at least one wall 55. The chamber/housing 50 comprises an inlet port 60 and outlet port 70. A tissue 10 can comprise a plurality of parenchymal cell islands interspersed with a stromal cell population. In one specific embodiment, the tissue or cellular array is defined by a specific distance between and/or size of the cellular island.

The top portion 30 of substrate 20 sealingly engages chamber/housing 50 to create a flow space (depicted by the arrows in FIG. 4). The chamber/housing 50 comprises openings for fluid flow. Fluid supply tubes are provided at the inlet 60 and are in fluid communication with gas exchanger 100, pump 90, and fluid reservoir 80. Return tubes are provided at the outlet 70. Fluid circulation is maintained in the system using a pump 90 that can be any pump routinely used in cell culture systems including, for example, syringe pumps and peristaltic or other type of pump for delivery of fluid through the bioreactor.

Inlet port 60 and outlet ports 70 comprise fittings or adapters that mate tubing to maintain circulation of the fluid in the system. The fittings or adapters may be a Luer fitting, screw threads, or the like. The tubing fittings or adapters may be composed of any material suitable for delivery of fluid (including nutrient media) for cell culture. Such tubing fittings and adapters are known in the art. Typically, inlet port 60 and outlet port 70 comprise fittings or adapters that accept tubing having a desired inner diameter for the size of the reactor and the rate of fluid flow.

Substrate 20 can be made of any material suitable for culturing mammalian cells. Although substrate 20 is depicted in FIG. 4 being a part of the bioreactor, it will be recognized that the substrate can be prepared for culture in the absence of the bioreactor. In one aspect, the substrate 20 can be a traditional tissue culture dish. For example, the substrate can be a material that can be easily sterilized such as plastic or other artificial polymer material, so long as the material is biocompatible. Substrate 20 can be any material that allows cells and/or tissue to adhere (or can be modified to allow cells and/or tissue to adhere) and that allows cells and/or tissue to grow in one or more layers. Any number of materials can be used to form the substrate 20 as described herein.

Certain materials, such as nylon, polystyrene, and the like, are less effective as substrates for cellular and/or tissue attachment. When these materials are used as the substrate it is advisable to pre-treat the substrate prior to inoculation with cells in order to enhance the attachment of cells to the substrate. For example, prior to inoculation with stromal cells and/or parenchymal cells, nylon substrates should be treated with 0.1M acetic acid, and incubated in polylysine, FBS, and/or collagen to coat the nylon. Polystyrene could be similarly treated using sulfuric acid.

Where the *in vitro* generated artificial tissue is itself to be implanted *in vivo,* a biodegradable substrate such as polyglycolic acid, collagen, polylactic acid or hyaluronic acid should be used. Where the tissues are to be maintained for long periods of time or cryo-preserved, non-degradable materials such as nylon, dacron, polystyrene, polyacrylates, polyvinyls, teflons, cotton, and the like, may be used.

After a tissue has been grown, it can be frozen and preserved. In one aspect, the tissue is preserved by reducing the temperature to about 4 °C. Where the tissue is to be cryopreserved, cryopreservative is added. Methods for cryopreserving tissue will depend on the type of tissue to be preserved and are well known in the art.

The micropatterned tissues comprising cellular islands of the disclosure can be used in a wide variety of applications. These include, but are not limited to, transplantation or implantation of the cultured artificial tissue *in vivo;* screening cytotoxic compounds, growth/regulatory factors, pharmaceutical compounds, and the like, *in vitro;* elucidating the mechanisms of certain diseases; studying the mechanisms by which drugs and/or growth factors operate; diagnosing and monitoring cancer in a patient; gene therapy and protein delivery; the production of biological products; and as the main physiological component of an extracorporeal organ assist device, to name a few. The tissues cultured by means of the bioreactors of the disclosure are particularly suited for the above applications, as the bioreactors allow the culturing of tissues having multifunctional cells. Thus, these tissues effectively simulate tissues grown in vivo.

In one embodiment, the tissue (*e.g.,* in a bioreactor) could be used *in vitro* to produce biological cell products in high yield. For example, a cell which naturally produces large quantities of a particular biological product (*e.g*. a growth factor, regulatory factor, peptide hormone, antibody, and the like) or a host cell genetically engineered to produce a foreign gene product could be cultured using the bioreactors of the disclosure *in vitro.*

For example, to use a bioreactor to produce biological products, a media flow having a concentration of solutes such as nutrients, growth factors and gases flows in through port 60 and out through port 70, over a tissue 10 seeded on substrate 20. The issue is designed with a desired cellular island size and/or distribution that promote the production of a biological product. The concentrations of solutes and nutrients provided are such that the tissue layer produces the desired biological product. Product is then excreted into the media flows, and can be collected from the effluent stream exiting through outlet port 70 using techniques that are well-known in the art .

As indicated above, reactors of different scales can be used for different applications. A large scale reactor can be used to study the effects of nutrient, drugs, and the like on tissue function (*e.g.,* ischemia on the liver and its implications such as cellular hypoxic response and organ preservation). A high throughput reactor can be used for the evaluation of drugs for metabolism, toxicity and adverse xenobiotic interactions. It could also be used for the evaluation of potential cancer drugs and other pharmacological agents in variable oxygen environments. For example, miniaturized bioreactor system can be made into an array such as depicted in FIG. 5.

For growth of cells including, for example, hepatocytes and/or stromal cells, media containing solutes required for sustaining and enhancing tissue growth are contacted with the cells. Solutes in the fluid media include nutrients such as proteins, carbohydrates, lipids, growth factors, as well as oxygen and other substances that contribute to cell and/or tissue growth and function. For example, the oxygen gas concentration in the bioreactor system can be regulated to maintain tissue morphology (*e.g*., zonation in liver tissue cultures). The solutes in the media as well as those produced and released by cells in culture facilitate the development of multifunctional cells.

In another aspect, the invention provides the use of a combination of modified oxygen delivery and micropatterning of co-cultures in order to optimize the tissue culture for specific physiologic functions including, for example, synthetic, metabolic, or detoxification function (depending on the function of interest) in hepatic cell cultures.

Typically, in practicing the methods of the disclosure, the cells are mammalian cells, although the cells may be from two different species (*e.g.*, pigs, humans, rats, mice, and the like). The cells can be primary cells, or they may be derived from an established cell-line. Although any cell type that adheres to a substrate can be used in the methods and systems of the disclosure (*e.g.*, parenchymal and/or stromal cells), exemplary combinations of cells for producing the co-culture include, without limitation: (a) human hepatocytes (*e.g*., primary hepatocytes) and fibroblasts (*e.g*., normal or transformed fibroblasts, such as NIH 3T3-J2 cells); (b) hepatocytes and at least one other cell type, particularly liver cells, such as Kupffer cells, Ito cells, endothelial cells, and biliary ductal cells; and (c) stem cells (*e.g.,* liver progenitor cells, oval cells, hematopoietic stem cells, embryonic stem cells, and the like) and human hepatocytes and/or other liver cells and a stromal cell (*e.g.*, a fibroblast). Other combination of hepatocytes, liver cells, and liver precursor cells.

In another aspect, certain cell types have intrinsic attachment capabilities, thus eliminating a need for the addition of serum or exogenous attachment factors. Some cell types will attach to electrically charged cell culture substrates and will adhere to the substrate via cell surface proteins and by secretion of extracellular matrix molecules. Fibroblasts are an example of one cell type that will attach to cell culture substrates under these condition.

Cells useful in the methods of the disclosure are available from a number of sources including commercial sources. For example, hepatocytes may be isolated by conventional methods (Berry and Friend, 1969, J. Cell Biol. 43:506-520) which can be adapted for human liver biopsy or autopsy material. Typically, a cannula is introduced into the portal vein or a portal branch and the liver is perfused with calcium-free or magnesium-free buffer until the tissue appears pale. The organ is then perfused with a proteolytic enzyme such as a collagenase solution at an adequate flow rate. This should digest the connective tissue framework. The liver is then washed in buffer and the cells are dispersed. The cell suspension may be filtered through a 70 µm nylon mesh to remove debris. Hepatocytes may be selected from the cell suspension by two or three differential centrifugations.

For perfusion of individual lobes of excised human liver, HEPES buffer may be used. Perfusion of collagenase in HEPES buffer may be accomplished at the rate of about 30 ml/minute. A single cell suspension is obtained by further incubation with collagenase for 15-20 minutes at 37 °C. (Guguen-Guillouzo and Guillouzo, eds, 1986, "Isolated and Culture Hepatocytes" Paris, INSERM, and London, John Libbey Eurotext, pp. 1-12; 1982, Cell Biol. Int. Rep. 6:625-628).

Hepatocytes may also be obtained by differentiating pluripotent stem cell or liver precursor cells (*i.e*., hepatocyte precursor cells). The isolated hepatocytes may then be used in the culture systems described herein.

Stromal cells include, for example, fibroblasts obtained from appropriate sources as described further herein. Alternatively, the stromal cells may be obtained from commercial sources or derived from pluripotent stem cells using methods known in the art.

Fibroblasts may be readily isolated by disaggregating an appropriate organ or tissue which is to serve as the source of the fibroblasts. This may be readily accomplished using techniques known to those skilled in the art. For example, the tissue or organ can be disaggregated mechanically and/or treated with digestive enzymes and/or chelating agents that weaken the connections between neighboring cells making it possible to disperse the tissue into a suspension of individual cells without appreciable cell breakage. Enzymatic dissociation can be accomplished by mincing the tissue and treating the minced tissue with any of a number of digestive enzymes either alone or in combination. These include, but are not limited to, trypsin, chymotrypsin, collagenase, elastase, and/or hyaluronidase, DNase, pronase, dispase and the like. Mechanical disruption can also be accomplished by a number of methods including, but not limited to, the use of grinders, blenders, sieves, homogenizes, pressure cells, or insonators. For a review of tissue disaggregation techniques, see Freshney, Culture of Animal Cells. A Manual of Basic Technique, 2d Ed., A. R. Liss, Inc., New York, 1987, Ch. 9, pp. 107-126.

Once the tissue has been reduced to a suspension of individual cells, the suspension can be fractionated into subpopulations from which the fibroblasts and/or other stromal cells and/or elements can be obtained. This also may be accomplished using standard techniques for cell separation including, but not limited to, cloning and selection of specific cell types, selective destruction of unwanted cells (negative selection), separation based upon differential cell agglutinability in the mixed population, freeze-thaw procedures, differential adherence properties of the cells in the mixed population, filtration, conventional and zonal centrifugation, centrifugal elutriation (counter-streaming centrifugation), unit gravity separation, countercurrent distribution, electrophoresis, fluorescence-activated cell sorting, and the like. For a review of clonal selection and cell separation techniques, see Freshney, Culture of Animal Cells. A Manual of Basic Techniques, 2d Ed., A. R. Liss, Inc., New York, 1987, Ch. 11 and 12, pp. 137-168.

The isolation of fibroblasts can, for example, be carried out as follows; fresh tissue samples are thoroughly washed and minced in Hanks balanced salt solution (HBSS) in order to remove serum. The minced tissue is incubated from 1-12 hours in a freshly prepared solution of a dissociating enzyme such as trypsin. After such incubation, the dissociated cells are suspended, pelleted by centrifugation and plated onto culture dishes. All fibroblasts will attach before other cells, therefore, appropriate stromal cells can be selectively isolated and grown. The isolated fibroblasts can then be used in the culture systems of the disclosure.

For example, and not by way of limitation, endothelial cells may be isolated from small blood vessels of the brain according to the method of Larson et al. (1987, Microvasc. Res. 34:184) and their numbers expanded by culturing *in vitro* using the bioreactor system of the disclosure. Silver staining may be used to ascertain the presence of tight junctional complexes specific to small vessel endothelium and associated with the "barrier" function of the endothelium.

Suspensions of pancreatic acinar cells may be prepared by an adaptation of techniques described by others (Ruoff and Hay, 1979, Cell Tissue Res. 204:243-252; and Hay, 1979, in, "Methodological Surveys in Biochemistry Vol. 8, Cell Populations." London, Ellis Hornwood, Ltd., pp.143-160). Briefly, the tissue is minced and washed in calcium-free, magnesium-free buffer. The minced tissue fragments are incubated in a solution of trypsin and collagenase. Dissociated cells may be filtered using a 20 µm nylon mesh, resuspended in a suitable buffer such as Hanks balanced salt solution (HBSS), and pelleted by centrifugation. The resulting pellet of cells can be resuspended in minimal amounts of appropriate media and inoculated onto a substrate for culturing in the bioreactor system of the disclosure. The pancreatic cells may be cultured with stromal cells such as fibroblasts. Acinar cells can be identified on the basis of zymogen droplet inclusions.

Cancer tissue may also be cultured using the methods and bioreactor culture system of the disclosure. For example, adenocarcinoma cells can be obtained by separating the adenocarcinoma cells from stromal cells by mincing tumor cells in HBSS, incubating the cells in 0.27% trypsin for 24 hours at 37 °C and further incubating suspended cells in DMEM complete medium on a plastic petri dish for 12 hours at 37 °C. Stromal cells selectively adhered, to the plastic dishes.

The tissue cultures and bioreactors of the disclosure may be used to study cell and tissue morphology. For example, enzymatic and/or metabolic activity may be monitored in the culture system remotely by fluorescence or spectroscopic measurements on a conventional microscope. In one aspect, a fluorescent metabolite in the fluid/media is used such that cells will fluoresce under appropriate conditions (*e.g.*, upon production of certain enzymes that act upon the metabolite, and the like). Alternatively, recombinant cells can be used in the cultures system, whereby such cells have been genetically Modified to include a promoter or polypeptide, that produces a therapeutic or diagnostic product under appropriate conditions (*e.g.*, upon zonation or under a particular oxygen concentration). For example, a hepatocyte may be engineered to comprise a GFP (green fluorescent protein) reporter on a P450 gene (CYPIA1)., Thus, if a drug activates the promoter, the recombinant cell fluoresces. This is useful for predicting drug-drug interactions that occur due to upregulation in P450s.

The various techniques, methods, and aspects of the invention described above can be implemented in part or in whole using computer-based systems and methods. For example, computer implemented methods can be used in lithography techniques to design cellular islands.

The working examples provided below are to illustrate, not limit, the disclosure. Various parameters of the scientific methods employed in these examples are described in detail below and provide guidance for practicing the disclosure in general.

In these particular working examples, hepatocytes are co-cultured with fibroblasts. Similar methods can be used to co-culture other combinations of cells. These experiments demonstrate that one or more cell types can be cultured in a bioreactor system with controlled oxygen to obtain cells that are phenotypically similar to corresponding cells *in vivo* as well as tissue that is morphologically similar to tissue *in vivo.* Although the invention has been generally described above, further aspects of the invention will be apparent from the specific disclosure that follows, which is exemplary and not limiting.

### EXAMPLES

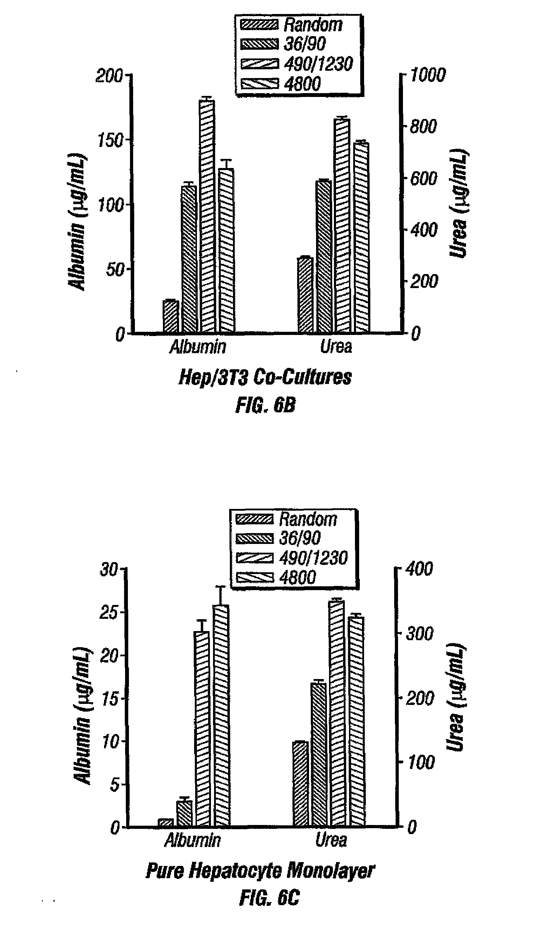

**Micropatterning of Collagen.** Elastomeric Polydimethylsiloxane (PDMS) stencil devices, consisting of thick-membranes (∼300µm) with through-holes (500µm with 1200µm center-to-center spacing) at the bottom of each well of a 24-well mold were provided by Surface Logix, Inc (Brighton, MA). Stencil devices were first sealed (via gentle pressing) to tissue culture treated polystyrene omnitrays (Nunc, Rochester, NY), then each well was incubated with a solution of type-I collagen in water (100µg/mL) for 1 hour at 37°C. Purification of collagen from rat-tail tendons was previously described. The excess collagen solution in each well was aspirated, the stencil was removed and a PDMS "blank" (24-well mold without stencil membranes) was applied. Collagen-patterned polystyrene was stored dry at 4°C for up to 2 weeks. In some cases, micropatterned collagen was fluorescently labeled via incubation (1 hour at room temperature) with Alexa Fluor® 48B carboxylic acid, succinimidyl ester (Invitrogen, Carlsbad, CA) dissolved in phosphate buffered saline (PBS) at 20µg/mL. For experiments in Fig. 6, collagen was micropatterned in various dimensions on glass substrates using conventional photolithographic techniques as described previously.

**Fibroblast Culture.** 3T3-J2 fibroblasts were the gift of Howard Green (Harvard Medical School)1. Cells were cultured at 37°C, 5% CO₂ in Dulbecoo's Modified Eagle's Medium (DMEM) with high glucose, 10% (v/v) calf serum, and 1% (v/v) penicillin-streptomycin. In some cases, fibroblasts were growth-arrested by incubation with 10µg/mL Mitomycin C (Sigma, St. Louis, MO) in culture media for 2 hours.

**Microscopy.** Specimens were observed and recorded using a Nikon Diaphot microscope equipped with a SPOT digital camera (SPOT Diagnostic Equipment, Sterling Heights, MI), and MetaMorph Image Analysis System (Universal Imaging, Westchester, PA) for digital image acquisition.

**Gene Expression Profiling.** Micropatterned hepatocytes-fibroblast co-cultures were washed 3 times with phosphate buffered saline (PBS) to remove traces of serum, followed by treatment with 0.05% Trypsin/EDTA (Invitrogen) for 3min at 37°C. Fibroblasts were much more sensitive to trypsin-mediated detachment than hepatocytes arranged in clusters (500µm) via micropatterning. Following incubation with trypsin, plates were shook mildly to remove loosely attached fibroblasts, the supernatant was aspirated and the attached hepatocytes (∼95% purity) were washed 3 times with serum-supplemented hepatocyte medium to neutralize and remove traces of trypsin from the cultures. Hepatocytes RNA was extracted via TRIzol (Invitrogen) and purified using the RNeasy kit (Qiagen) as per manufacturers' instructions. The RNA was labeled, hybridized to an Affymetrix (Santa Clara, CA) Human U133 Plus 2.0 Array, and scanned as described previously. Briefly, double-strand cDNA was synthesized using a T7- (dt)24 primer (Oligo) and reverse transcription (Invitrogen) cDNA was then purified with phenol/chloroform/isoamyl alcohol in Phase Lock Gels, extracted with ammonium acetate and precipitated using ethanol. Biotin-labeled cRNA was synthesized using the BioArray™ HighYield™ RNA Transcript Labeling Kit, purified over RNeasy columns (Qiagen), eluted and then fragmented. The quality of expression data was assessed using the manufacturer's instructions which included criteria such as low background values and 3' /5' actin and GAPDH (Glyceraldehyde-3-phosphate dehydrogenase) ratios below 2. All expression data was imported to GCOS (GeneChip Operating System vl.2) and scaled to a target intensity of 2500 to enable comparison across conditions.

**Phrase I & II Enzyme Activity Assays.** Chemicals were purchased from Sigma: Coumarin (CM), 7-Hydroxycoumarin (7-HC), Ethoxyresorufin (ER), Resorufin (RR), Ketoconazole (KC), Sulfaphenazole (SP), Methoxsalen (MS) Salicylamide (SC) or purchased from BD-Gentest: 7-methoxy-4-trifluoromethylcoumarin (MFC), 7-benzyloxy-4-trifluoromethylcoumarin (BFC), 7-hydroxy-4-trifluoromethylcoumarin (7-HFC). Cultures were incubated with substrates (CM, MFC, BFC at 50µM, ER at 8µM, 7-HC at 100µM) dissolved in DMEM without phenol red for 1 hour at 37°C. For inhibition studies, cultures were incubated with substrates in the presence of specific inhibitors (MS at 25µM with CM, SP at 50µM with MFC, KC at 50µM with BFC, SC at 3mM with 7-HC). The reactions were stopped by collection of the incubation medium. Then, potential metabolite conjugates formed via Phase II activity were hydrolyzed by incubation of supernatants with ß-glucuronidase/arylsulfatase (Roche, IN) for 2 hours at 37° C. Samples were diluted 1:1 in quenching solution and fluorescent metabolite formation was quantified by means of a fluorescence micro-plate reader (Molecular Devices, Sunnyvale, CA) as described elsewhere in detail. Production of 7-HC from CM is a reaction (CM 7-Hydroxylation) mediated by CYP2A6 in humans, production of 7-HFC from BFC or MFC (dealkylation) is mediated by several different CYP450s, and production of RR from ER (ER Odealkylation) is mediated by CYPIA2. Conjugation of 7-HC with glucuronic acid and sulfate groups is mediated by Phase II enzymes, UPD-Glucuronyl-transferase and Sulfotransferase, respectively.

Hepatocyte Isolation and Culture. Primary rat hepatocytes were isolated from. 2-3-month old adult female Lewis rats (Charles River Laboratories, Wilmington, MA) weighing 180-200g. Detailed procedures for rat hepatocyte isolation and purification were previously described. Routinely, 200-300 million cells were isolated with 85%-95% viability and >99% purity. Hepatocyte culture medium consisted of Dulbecco's Modified Eagle's medium (DMEM) with high glucose, 10% (v/v) fetal bovine serum, 0.5 U/mL insulin, 7 ng/mL glucagon, 7.5 µg/mL hydrocortisone, and 1% (v/v) penicillin-streptomycin. Primary Human hepatocytes were purchased in suspension from vendors permitted to sell products derived from human organs procured in the United States of America by federally designated Organ Procurement Organizations. Hepatocytes vendors included: In vitro Technologies (Baltimore, MD), Cambrex Biosciences (Walkersville, MD), BD Gentest (Woburn, MA), ADMET Technologies (Durham, NC), CellzDirect (Pittsboro, NC) and Tissue Transformation Technologies (Edison NJ). All work was done with the approval of COUHES (Committee on use of human experimental subjects). Upon receipt human hepatocytes were pelleted via centrifugation at 50xg for 5min (4°C). The supernatant was discarded, cells were re-suspended in hepatocyte culture medium, and viability was assessed using trypan blue exclusion (70-90%).

**Hepatocyte-Fibroblast Co-Cultures.** In order to create micropatterned co-cultures, hepatocytes were seeded in serum-free hepatocyte medium on collagen-patterned substrates, resulting in a hepatocytes pattern due to selective cell adhesion. The cells were washed with media 2 hours later to remove unattached cells and incubated with serum-supplemented hepatocyte medium overnight. 3T3-J2 fibroblasts were seeded in serum-supplemented fibroblasts medium 12-24 hours later to create co-cultures. Culture medium was replaced to hepatocyte medium 24 hours alter fibroblast seeding and subsequently replaced daily. For randomly distributed cultures, hepatocytes were seeded in serum-supplemented hepatocyte medium on substrates (glass or polystyrene) with a uniform coating of collagen. In some cases, hepatocytes were fluorescently labeled via incubation (1 hour at 37°C) with Calcein-AM (Invitrogen) dissolved in culture media at 5µg/mL. Fibroblasts were fluorescently labeled with CellTracker (Orange CMTMR, Invitrogen) as per manufacturer's instructions.

In one aspect, a bioreactor as depicted in Figure 5A-B was validated by analyzing the: dose response to acetometaphen in the bioreactors. The device was seeded with hepatocytes and J2-3T3 fibroblasts and allowed to stabilize for 5 days under static culture conditions. Next, the device was incorporated into the perfusion circuit and each of the eight bioreactors was exposed to a different concentration of acetometaphen generated by the microfluidic dilution tree (as shown in Figure 5). After 1 day of acetometaphen exposure, the viability was assessed through a: MTT stain and the intensity was measured in each bioreactor and plotted as a function of bioreactor acetometaphen concentration. The TD₅₀ was determined to be 13 mM for these perfusion studies which is the same values as found with the large flat plate bioreactor. These experiments were preformed under 21% oxygen, and the next step is to repeat these studies under different oxygen tensions. The bilayer delivery of oxygen was validated through experimental measurement of the oxygen concentration using ruthenium coated substrates (ruthenium's fluorescence is correlated with the oxygen concentration), and theoretically through the development of a multiphysics model coupling perfusion with diffusion. In addition fluorescence staining can be implemented in the bioreactors as shown by intracellular albumin and nuclear staining.

**Biochemical Assays.** Spent media was stored at -20°C. Urea concentration was assayed using a colorimetric endpoint assay utilizing diacetylmonoxime with acid and heat (Stanbio Labs, Boerne, TX). Albumin content was measured using enzyme linked immunosorbent assays (MP Biomedicals, Irvine, CA) with horseradish peroxidase detection and 3,3',5,5''-tetramethylbenzidine (TMB, Fitzgerald Industries, Concord, MA) as a substrate.

**Cytochrome-P450 Induction.** Stock solutions of prototypic CYP450 inducers (stigma) were made in dimethylsulfoxide (DMSO), except for Phenobarbital, which was dissolved in water. Cultures were treated with induces (Rifampin 25µM, ß-Naphthoflavone 30µM or 50µM, Phenobarbital 1mM, Omeprazole 50µM) dissolved in hepatocyte culture medium for 4 days. Control cultures were treated with vehicle (DMSO) alone for calculations of fold induction. To enable comparisons across inducers, DMSO levels were kept constant at 0.06% (v/v) for all conditions.

**Toxicity Assays.** Cultures were incubated with various concentrations of compounds dissolved in cultures medium for 24 hours (acute toxicity) or extended time periods (chronic toxicity, 1-4 days). Cell viability was subsequently measured via the MTT (3-(4,5-dimethylthiazol-2-yl)-2,5-diphenyl tetrazolium bromide; Sigma) assay, which involves cleavage of the tetrazolium ring by mitochondrial dehydrogenase enzymes to form a purple precipitate. MTT was added to cells in DMEM without phenol red at a concentration of 0.5mg/mL. After an incubation time of 1 hour, the purple precipitate was dissolved in a 1:1 solution of DMSO and Isopropanol. The absorbance of the solution was measured at 570nm (SpectraMax spectrophotometer, Molecular Devices, Sunnyvale, CA).

**statistical Analysis.** Experiments were repeated at least 2-3 times with duplicate or triplicate samples for each condition. Data from representative experiments is presented, whereas similar trends were seen in multiple trials. All error bars represent standard error of the means.

Furthermore, without induction, both CYP2B and CYP3A protein was present at low levels after 48 hour perfusion with little distinguishable spatial heterogeneity as compared to not detectable protein under static culture conditions. Next, induction of static cultures with phenobarbital (PB) over the same time period resulted in moderate CYP2B expression and low CYP3A. Dramatic expression of both CYPs over controls was seen after only 36 hours when cultures were perfused with PB. Though expression of CYP2B was increased in all regions, levels were highest in the lower-oxygen outlet regions. Similarly, CYP3A protein showed increasing expression from inlet to outlet. Based on previous studies that showed repression of PB-induced CYP2B expression by epidermal growth factor (EGF), added 2 nM EGF to the perfusion media. At a dose of 200 µM PB, EGF did not significantly alter CYP2B levels along the length of the chamber though maximal levels were noted in the outlet regions. CYP3A levels in response to PB and EGF also showed little difference from PB-only perfusion displaying maximal expression at the outlet.

Experiments were also carried out to evaluate dexamethasone (DEX) as an inducer of CYPs in this perfusion system. DEX induced CYP2B to high levels which were localized to inlet regions of the culture. For CYP3A, induction was mostly uniform, but not detectable in the outlet region. When EGF was added to DEX-perfused cultures, a significant shift in CYP2B spatial distribution was noted from inlet regions to the outlet. CYP3A induction remained uniform in response to DEX and EGF, but was extend across all regions of the culture.

Acetaminophen (APAP) was evaluated for its acute toxic effect on hepatocyte cultures and co-cultures (Figure 7 and 8; Static toxicity dose response of APAP). Viability, as assessed by MTT, decreased in a dose-dependant manner with reduced viability of 5% in hepatocytes. alone and 28% in co-culture at 40 mM APAP after 24 hours. These data suggested that a dose range from 0 - 20 mM APAP would result in moderate toxicity in bioreactor cultures. Figure 9 shows a panel of images of the full length (∼5.6 cm) of the bioreactor cultures perfused with various concentrations of APAP for 24 hours and then incubated with MTT. The presence and intensity of purple precipitate is proportional to cell viability. Of note is the dramatic decrease in staining from the inlet to the outlet region at a dose of 15 mM APAP as compared to control (moderate decrease) and 20 mM (no staining).

For further quantification of regional variations in viability, bright-field images were acquired at low magnification (40x) along the length of the culture for measurement of mean optical density (Figure 9). Under the control condition, viability decreased 30% from inlet to cutlet. However, at 10mM APAP, toxicity was more uniform over the culture but was decreased to 80% of average control viability. Administration of 15 mM APAP resulted in maximal toxicity in the outlet region, decreased 70% from the inset region. At the highest dose, 20 mM, toxicity was virtually complete.

Many members of the CYP superfamily responsible for phase I drug and steroid biotransformation are expressed in a zonal pattern in *vivo.* Among the determinants of the pericentral localization of CYPs under both normal and induced conditions are gradients of oxygen, nutrients, and hormones. Recapitulation of these dynamic gradients in bioreactor cultures resulted in spatial distributions of both CYP2B and CYP3A that mimic those found in vivo. Additionally CYP induction was potentiated by the perfusion microenvironment of the reactor as shown by the dramatic increase in protein levels over static cultures in response to 200 µM PB. Previous studies demonstrated that the repressive effects of EGF on PB induction are modulated by oxygen.

Addition of EGF with PB in the current study did not significantly alter the spatial CYP2B pattern, but in conjunction with DEX, EGF shifted maximal CYP2B expression from the inlet to the outlet. This shifting effect, also noted to a leaser extent in CYP3A expression, may be due the formation of EGF gradients, thus demonstrating how dynamic gradients of growth factors and hormones regulate CYP zonation.

The proposed mechanism of APAP hepatotoxicity involves the formation of a reactive intermediate, NAPQI, which initiates free-radial damage of intracellular structures. Toxic effects in this study are likely due to the depletion of glutathione, which provides protective inactivation of NAPQI. Though pericentral localization of APAP toxicity in vivo has been attributed to local expression of CYP isoenzymes 2E1 and 3A, reduced oxygen availability in centrilobular regions may also contribute by deleting ATP and glutathione, or increasing damage by reactive species . A combination of these factors likely resulted in the regional toxicity observed in reactor cultures under dynamic oxygen gradients. Demonstration of zonal toxicity in vitro allows decoupling of the effects of CYP bioactivation and glutathione levels on acute APAP tonicity.

Furthermore, this system may allow elucidation of the actions various clinically important compounds such as ethanol or N-acetyl-cysteine and their respective exacerbating or protective effects on APAP toxicity.

As demonstrated by the data, oxygen gradients were applied to cultures of rat hepatocytes to develop and in vitro model of liver zonation. Cells experienced oxygen conditions ranging from normoxia to hypoxia without compromising viability as shown by morphology and fluorescent markers of membrane integrity. The hepatocytes exposed to oxygen gradients exhibited characteristics of in *vivo* zonation upon induction as shown by PEPCK (predominantly upstream) and CYP2B (predominantly downstream) protein levels. With this *in vitro* model off liver zonation, the microenvironmental conditions seen in the liver sinusoid that are thought to be responsible for heterogeneous distribution of metabolic and detoxifying functions can be reproduced.

Cell seeding conditions and cell height can be kept uniform within the bioreactor system to insure uniformity of the flow field. The bioreactor experiments carries out in the specific examples herein, were typically conducted at a flow rate of 0.5 mL/min, corresponding to a shear stress of 1.25 dyne/ cm2 , although higher stress near 7.5 dynes/cm² may have been present at higher flow rates using validation experiments.

Liver-specific functions in human cocultures can be optimized by varying homotypic and heterotypic interactions. A photolithographic cell patterning technique is provided by the invention which allows study of the relative role of homotypic (hepatocyte-hepatocyte) and heterotypic (hepatocyte-fibroblast) cell-cell interactions in stabilization of liver-specific functions *in vitro* (see Figure 10). In order to study the role of heterotypic interactions on phenotypic functions of rat hepatocytes in cocultures, the experimental design shown in Figure 11 was used, which varies the size of the hepatocyte islands from single cell islands (36µm) to large circular colonies (17,800µm). Using this design, cocultures, were conducted in which the heterotypic interface varied over three orders of magnitude as estimated by image analysis; however, the ratio of cell populations, as well as total number of cells remained constant. In contrast, in conventional culture conditions, cell-cell interactions are varied by seeding density which is, in turn, coupled to both cell number and ratio of cell populations. As expected, liver functions (albumin and urea secretion) were upregulated in all co-cultured configurations compared to hepatocytes alone. However, the degree of upregulation varied with the micropatterned geometry. Rat co-cultures with a larger initial heterotypic interface (i.e. single cell islands) had highest levels of liver-specific functions, while only a modest upregulation was seen for the two patterns with the largest island sizes (see Figure 12). Hepatocytes in smaller patterns (<250 µm) intermingled significantly, whereas larger islands assumed a relatively stable conformation (∼weeks). Despite the tendency for some spatial configurations to reorganize, the 'initial' cellular microenvironment was found to have significant long-term effects on liver-specific functions.

Due to data showing that rat cocultures can be functionally optimized using micro-patterning, it was hypothesized that a similar optimization can be obtained for human cocultures as well. In order to compare results between species, similar pattern geometries were used to evaluate micropatterned human cocultures. Conventional cocultures contain hepatocytes in a random variety of island sizes from single cell islands to large aggregates (random cocultures). Hence, the expectation was that with a wide array of island sizes, function in random human cocultures would be at a level intermediate of the single island (36µm) and the large island (4,800µm) micropatterned configurations. However, micropatterned human cocultures (all configurations) reproducibly outperform (by several fold) their randomly distributed counterparts, which contain similar cell ratios and numbers (see Figure 13). Though the mechanism underlying such differences remains un-elucidated, the results indicate the advantage of micropatterning in obtaining highly functional *in vitro* human liver tissues. The previously published studies with rat cocultures showed that reducing hepatocyte island size while increasing heterotypic interactions (hepatocyte to fibroblast) led to greater hepatocyte function. In contrast to rat cocultures, human cocultures have a different architectural dependence, in that there is a functionally optimal micropatterned configuration (490µm islands) with its proper balance of homotypic (hepatocyte to hepatocyte) and heterotypic interactions. Also, micro-patterning human hepatocytes without fibroblast produced higher levels of albumin and urea than those seeded randomly. Specifically, in pure hepatocyte monolayers, 490µm and 4800µm islands provided for higher functions than the single cell array (36µm), figure 13. Optimization of liver-specific functions in human hepatocyte cultures/co-cultures via micro-patterning. Micropatterned cultures/cocultures performed better than randomly seeded ones. 'Random' indicates randomly seeded cultures, '36/90' indicates 36µm islands separated by 90µm center-to-center spacing, 490/1230 indicates 490µm islands separated by 1230µm center-to-center spacing, and 4.8mm indicates 7 x 4.8mm islands packed in a hexagonal array (although one of skill in the art will recognize that other array shapes may be used). These dimensions were chosen to keep the ratio of two cell types and total cell numbers constant. Graphs show hepatocyte function for a representative day 7, while trends were observed for several days. Micrographs of micropatterned cocultures, are shown in which hepatocyte islands are surrounded by 3T3-J2 fibroblasts.

Such a trend is consistent with the literature, in which phenotypic stabilization of human hepatocytes occurs more effectively with increasing levels of homotypic interactions. As with rat cocultures, the 36µm human hepatocyte islands reorganized within a day, thereby dissipating the pattern. The micro-patterns with the two large island sizes, however, were intact for the duration of the cultures (3 weeks). Thus, the 'optimal' micropatterned configuration was identified as 490µm islands with 1230 µm center-to-center spacing and a 3:1 fibroblast to hepatocyte ratio. Since reorganization of hepatocyte islands in this optimal configuration is minimal over the length of the culture, real-time tracking of individual islands for morphological and functional changes can be performed using specific reporter systems (i.e. green fluorescent protein).

Use of optimized micropatterned human cocultures to evaluate metabolism and toxicity of xenobiotics. In order to demonstrate that the optimized micropatterned human cocultures are effective, liver models for screening of drug candidates acute and chronic toxicity assays were conducted. Acute toxicity tests involved incubating eight day old cocultures for 24hrs with various drugs, some with known clinical hepatotoxic potential. After the incubation time period, the cell culture medium was aspirated and a viability assay was conducted (MTT assay - commercially available). Shown as part of Figure 14 are concentrations at which each of the drugs led to a reduction in 50% of the viability signal (LD₅₀ value) as compared to drug-free control cocultures. As can be seen, there are quantitative differences in the LD₅₀ values for clinical drugs and a heavy metal toxin, Cadmium chloride (CdCl₂) . Acetaminophen (APAP), which is the active ingredient in Tylenol, is commonly used clinically and is not toxic in the in vitro system, except at very high relative concentrations (35mM). Cadmium, on the other hand, is a severely toxic metal and such can be seen *in* vitro as well. Troglitazone, shown here to be severely toxic as compared to APAP, is the active ingredient in the drug Rezulin as a therapy for type 2 diabetes. Rezulin was withdrawn from the market voluntarily by its manufacturer in March of 2000 due to deaths related to liver problems.

Current *in* vitro human liver models typically rely on hepatocytes in suspension or as pure monolayer on collagen. Hepatocytes are attachment-dependent cells and thus the 'cells in suspension' model is only viable for a few hours, while the pure hepatocyte monolayer typically loses viability and liver-specific function within 24 hrs. Thugs, chronic toxicity assays in which the cells are incubated repeatedly for several days or weeks with low doses of a drug cannot be conducted in current liver model systems. Since the optimized micropatterned human cocultures, routinely last for 3 weeks, chronic toxicity tests can be performed. In figure 7 is shown chronic toxicity in human cocultures when they are incubated with acetaminophen repeatedly every 24hrs for several days. As can be seen, the viability of cocultures, drops off significantly as a function of time. One particular advantage of the 2-D system is that cellular morphology can be easily monitored as compared to complicate 3-D systems. The hepatocyte morphology after only a 24hr incubation with 30mM APAP shows drastic changes when compared to the drug-free control, in which hepatocytes, look normal (Figure 7). Even though the morphology has been altered significantly, the viability for 30mM APAP incubation is close to 90%. Therefore, cellular morphology can serve as an additional indicator of toxicity or unwanted cellular changes due to a drug.

Besides toxicity testing, induction and inhibition of CYP450 enzymes is quite common during the in *vitro* testing of a new drug candidates. As can be seen in Figure 15, commercially available fluorescent substrates (BD Gentest) were used to demonstrate induction and inhibition of specific CYP450 enzymes in the optimized micropatterned human cocultures. Specifically, tests were conducted for CYP3A4, 1A2 and 2C9, three major human CYP450s, using 7-Benzyloxy-4-(trifluoromethyl)-coumarin (BFC), 7-Methoxy-4-(trifluoromethyl)-coumarin (MFC) and ethoxy-resorufin as substrates respectively. Both BFC and MFC are cleaved specifically by CYP450 enzymes into 7-Hydroxy-4-(trifluoromethyl)-coumarin (HFC), which is a fluorescent compound whose fluorescence can be quantified using a fluorimeter. Ethoxy-resorufin gets cleaved by CYP1A2 into fluorescent resorufin. Cocultures were treated with classic inducers in cell culture medium (Rifampin for 3A4 and 2C9 and Beta-napthoflavone for 1A2) for 72hrs to upregulate specific CYP450 intracellular levels. The inducer was then removed and the cells were incubated with substrates for ½ - 1hr. For inhibition assays, induced cocultures, were incubated with the substrate along with a known specific inhibitor for each CYP450 of interest (i.e. Ketoconazole inhibits CYP3A4). As can be seen, induction and inhibition were effective with all the tested drugs, suggesting that major CYP450 enzymes are active in the cocultures.

One of the major concerns facing current *in vitro* liver models is the rapid (hrs) decline of expression levels (RNA) of important liver-specific genes. Thus, expression levels of important liver-specific genes in the optimized micropatterned cocultures, were compared to those in conventional pure hepatocyte monolayers after several days of culture. Using DNA microarrays (Affymetrix GeneChips), the data demonstrate as shown in Figure 16 that optimized micropatterned co-cultured human hepatocytes have relatively high expression levels of many important phase I and phase II drug metabolism genes even at day 6 of culture as compared with pure hepatocytes on collagen. In order to obtain purified hepatocyte from cocultures, 0.05% Trypsin/EDTAwas used to selectively detach fibroblasts from the substrate. Such a selective release provided over 90% hepatocyte purity for GeneChip analysis.

Drawing photolithographic micropatterning techniques to manipulate functions of rodent hepatocytes upon co-cultivation with stromal cells, a microtechnology-based process utilizing elastomeric stencils to miniaturize and characterize human liver tissue in an industry-standard multiwell format was used. The approach incorporates 'soft lithography,' a set of techniques utilizing reusable, elastomeric, polymer (Polydimethylsiloxans-PDMS) molds of microfabricated structures to overcome limitations of photolithography. In one aspect, the invention provides a process using PDMS stencils consisting of 300µm thick membranes with through-holes at the bottom of each well in a 24-well mold (Fig. 13a). To micropatternall wells simultaneously, the assembly was sealed against a polystyrene plate. Collagen-1 was physisorbed to exposed polystyrene, the stencil was removed, and a 24-well PDMS 'blank' was applied. Co-cultures were 'micropatterned' by selective adhesion of human hepatocytes to collagenous: domains, which were then surrounded by supportive murine 3T3-J2 fibroblast. The diameter of through-holes determined the size of collagenous domains and thereby the balance of homotypic (hepatocyte/hepatocyte) and heterotypic (hepatocyte/stroma) interactions in the microscale tissue.

Collagen island diameter was varied over several orders-of-magnituds. Hepatocyte clustering consistently improved liver-specific functions when compared to unorganized co-cultures (Fig. 6). Furthermore, hepatocellular function was maximal for configurations containing ∼500µm islands with ∼1200µm spacing. These findings are consistent with rodent data in that 3T3 fibroblasts were able to stabilize hepatocellular functions across both species; however, human hepatocytes were more dependent on homotypic interactions than rat hepatocytes . Thus, the microscale human liver tissue developed and characterized herein represents 24-well plates with each well containing ∼10,000 hepatocytes organized in 37 colonies of 500µm diameter, for a total of 888 repeating hepatic microstructures per plate (Fig. 3b).

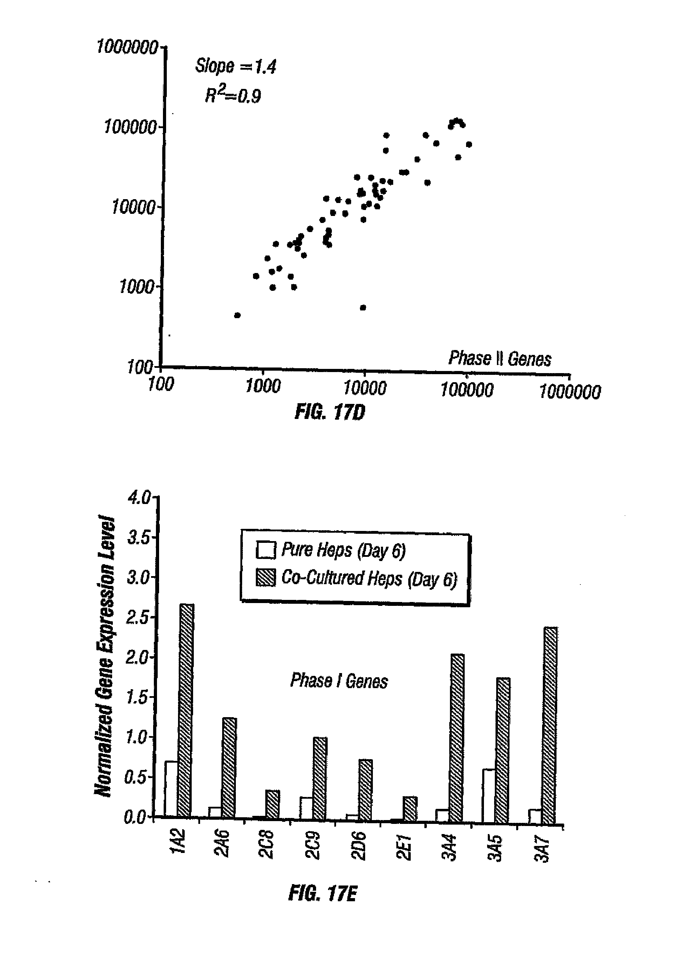

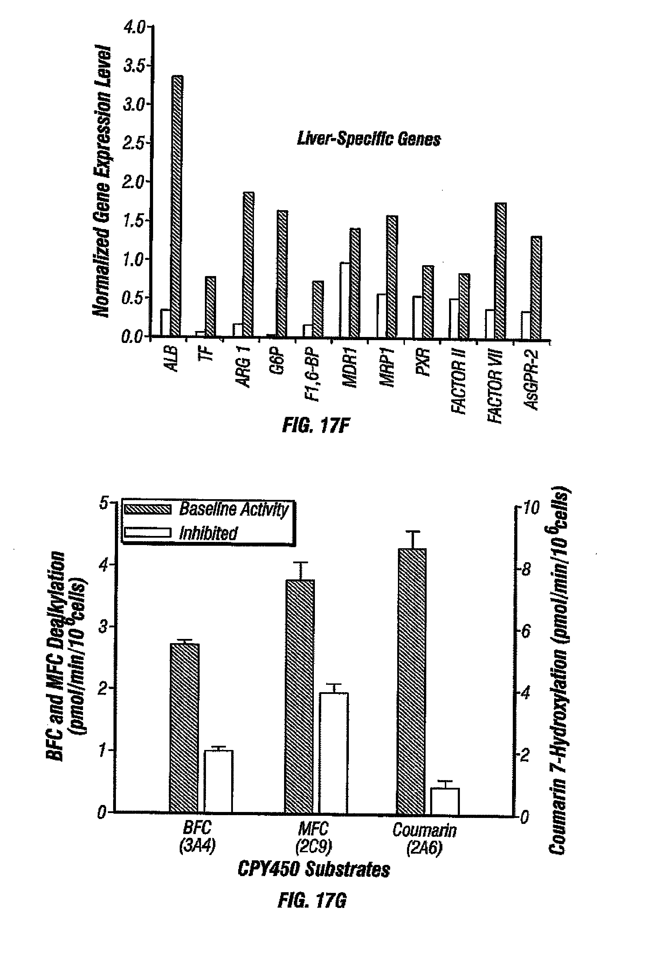

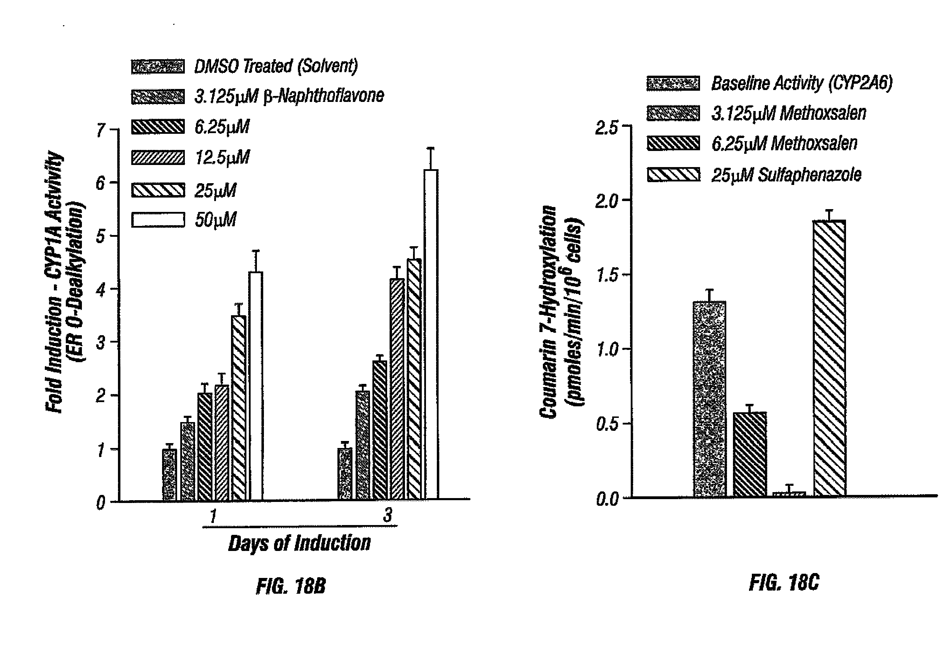

In order to qualitatively assess the stability of the microscale human liver tissues, hepatocyte morphology and persistence of microscale organization were monitored and found to be maintained for duration of the culture, typically 3-6 weeks (Fig. 3c). To quantitatively assess the stability of liver-specific functions, albumin and urea secretions were measured. Both markers were stable for several weeks in the platform (Fig.17a-b), whereas a monotonic decline was confirmed in unorganized pure hepatocyte cultures (Fig. 18). To obtain a more global perspective, gene expression profiling on human hepatocytes from 1-week old microscale tissues (day 6) via selective trypsinization to remove fibroblasts (∼95% purity, see supplemental methods online). The ability to obtain purified hepatocyte RNA from co-cultures is enhanced by clustering via micropatterning and is advantageous for genomic applications (e.g. toxicogenomics). For comparison, gene expression of fresh, unorganized, pure hepatocytes (12hr after plating, day 1) considered to be the 'gold standard' and unorganized pure hepatocytes on day 6 were characterized as their liver-specific functions declined. Global scatter plot comparison revealed that gene expression intensities in hepatocytes from 1-week old microscale tissues were similar to intensities in pure hepatocytes on day 1 as assessed by the slope (0.96) of a least-squares linear fit (Fig. 17c). Furthermore, phase-II metabolism genes in hepatocytes from microscale tissues were expressed at levels similar to those in pure hepatocytes on day 1 (Fig. 17d). The expression of cytochrome-P450 (CYP450) genes were significantly down-regulated in pure hepatocytes by day 6, whereas hepatocytes in the platform retained expression at high levels (Fig. 17e). Similar trends were seen for genes from diverse pathways of liver-specific functions such as gluconeogenesis, drug transporters, coagulation factors and cell surface receptors (Fig. 17f).

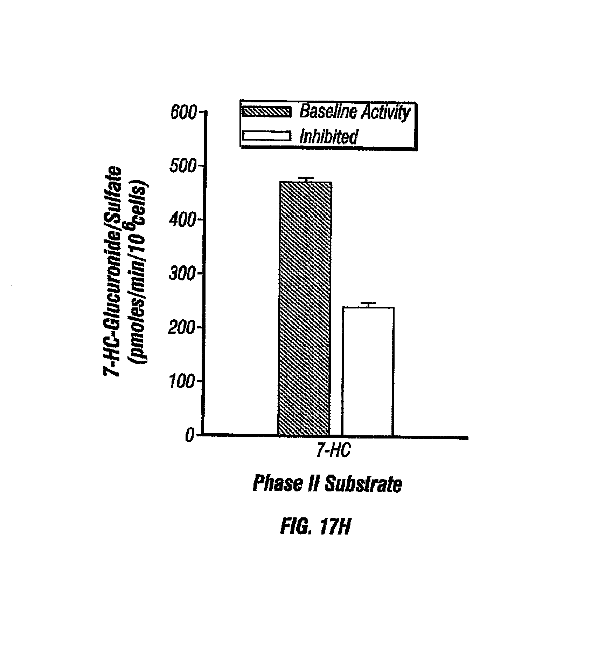

In order to assess utility of the microscale human liver tissue for drug metabolism studies, CYP450 activity, drug-drug interactions, and phase-II metabolism was characterized. CYP450 activity was assessed using fluorescent substrates and found to be retained in untreated microscale tissues (Fig. 17g). Such 'baseline' activity is critical for evaluation of metabolism-mediated mechanisms of toxicity. Competition for specific CYP450 enzymes was preserved in the platform as indicated by decreased substrate metabolism upon treatment with inhibitors. Phase-II activities (glucuronidation/sulfation) and their inhibition via prototypic compounds were also retained as determined by conjugation of 7-hydroxycoumarin (Fig. 17h).

To assess utility of the microscale human liver tissue for toxicity assays, the acute and chronic toxicity of model hepatotoxins were quantified. Compounds where characterized by their TC₅₀, defined as the concentration which produced 50% reduction in mitochondrial activity after 24hr exposure (Fig. 19a). Relative toxicity corresponded to relative hepatotoxicity of these compounds in humans. For example, the TC₅₀ for troglitazone (oral hypoglycemic withdrawn due to hepatotoxicity) was two orders of magnitude lower than acetaminophen (over-the-counter analgesic). Importantly, relative toxicity of compounds in the same class such as troglitazone and its FDA-approved analogues, rosiglitazone and pioglitazone also corresponded to clinical reports. Established mechanisms of toxicity could also be inferred from toxicity profiles in the platform. For instance, cadmium showed a relatively linear toxic profile while acetaminophen exhibited a toxicity 'shoulder' consistent with glutathione depletion (Fig. 18). Establishment of liver tissue that is stable over several weeks is crucial for evaluating chronic toxicity due to repeated exposures. In Figure 19b, the invention demonstrates dose and time-dependent toxicity of acetaminophen. Concentrations that were not lethal at 24hr caused extensive cell death after prolonged exposure. Furthermore, morphologic changes were readily observed prior to cell death, allowing the potential to detect sub-lethal toxicity at lower concentrations than those required for frank cell death.

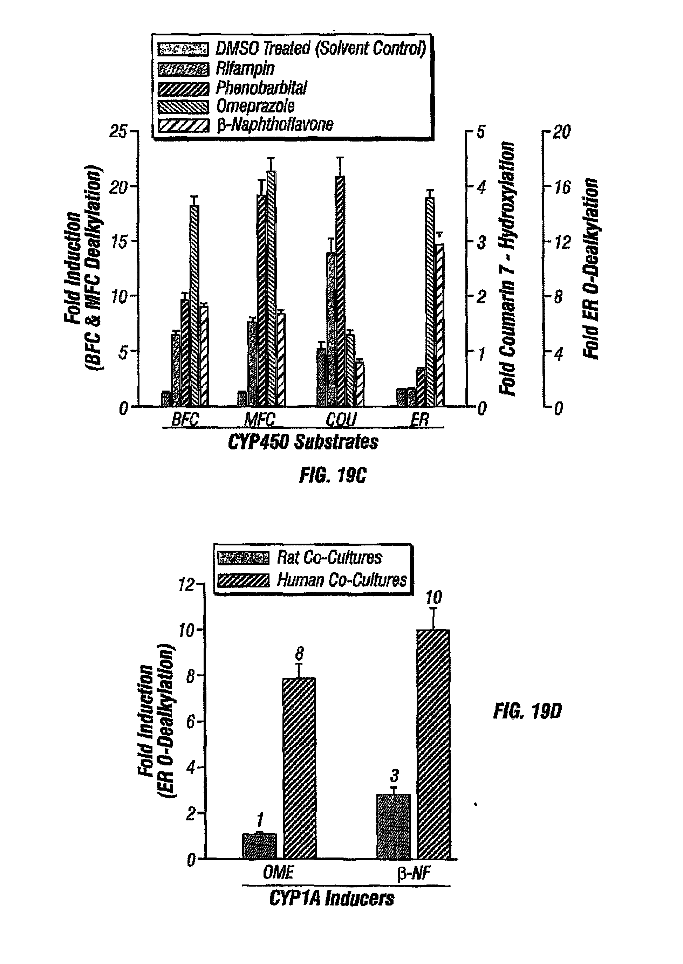

Induction of CYP450 activity in the microscale human liver tissues was demonstrated using prototypic inducers and fluorescent substrates (Fig. 19c). For example, CYP2A6 induction was observed upon treatment with Rifampin and Phenobarbital, while Omeprazole and β-Naphthoflavone had weaker effects, consistent with the literature. A reverse trend was seen for CYP1A2 induction. Modulation of CYP450 activities depends on both the dose and time of exposure to compounds. In Fig. 18, β-Naphthoflavone is shown to induce CYP1A2 activity in a dose and time-dependent manner in the platform, while methoxsalen shows dose-dependent inhibition of CYP2A6 activity. Furthermore, species-specific differences in induction was demonstrated by comparing the responses of microscale human and rat liver tissues. Omeprazole, reported to be a more effective inducer of human CYP1A2 than rat CYP1A22, was B-fold more effective in human over rat models (Fig. 19d).

An advantageous feature of the platform of the invention is its modular design in that various liver or non-liver derived stroma can be used to surround hepatocyte colonies/islands to form micropatterned tissues. 3T3 fibroblasts were chosen because of their ready availability, ease of propagation, and evidence showing that this immortalized cell line can induce high levels of liver-specific functions. Nonetheless, to demonstrate versatility of the platform, co-cultivates of micropatterned human hepatocytes with the non-parenchymal fraction of the human liver also demonstrated stabilization of hepatocyte functions. Furthermore, stencils were used to create a co-culture model of the rat liver that remains functional for over 2 months, allowing chronic studies to be conducted on hundreds of identical rodent liver tissues, thereby reducing noise arising from animal-to-animal variability (Fig. 20).

The invention demonstrates that micropatterned clusters of human hepatocytes outperformed their randomly distributed counterparts by several fold, consistent with reports that confluent hepatocyte cultures retain liver-specific functions better than sparse cultures, partly through cadherin interactions. Subsequent introduction of non-parenchymal cells further enhanced hepatocellular functions and longevity of the liver tissues. Thus, the microscale platform described herein uses an order-of-magnitude fewer hepatocytes (10K vs. 200K) and maintains phenotypic functions for a longer time than conventional pure monolayers (weeks vs. days) in similar multiwell formats. Given the high cost of human hepatocytes, (∼$80/million), such advantages represent a significant cost savings. The platform, demonstrates induction of liver-specific functions in fresh hepatocytes across donors of multiple age groups, sexes and medical histories (Table 1). The cultures were also capable of being successfully cryopreserved similar to those now widely utilized for short-term cultures, thus providing the potential to generate microscale liver tissue on demand.

| **Donor#** | **Age (years)** | **Sex** | **Cause of Death** | **Vendor** |
|---|---|---|---|---|
| 1 | 4 | N/A | Anoxia | ADMET |
| 2* | 5 | M | Anoxia | BD-Gentest |
| 3 | 7 | F | N/A | Cambrex |
| 4 | 14 | F | Gun shot wound | ADMET |
| 5 | 19 | M | Motor vehicle accident | In Vitro Technologies |
| 6 | 20 | M | Gun shot wound | In Vitro Technologies |
| 7 | 52 | M | Aortic dissection | In Vitro Technologies |
| 8 | 53 | M | Brain stem hemorrhage | Tissue Transformation Technologies |
| 9 | 54 | F | Cardiac arrest | In Vitro Technologies |
| 10 | 55 | M | Seizure | Tissue Transformation Technologies |
| 11 | 60 | M | N/A | CellzDirect |
| 12 | 61 | M | Motor vehicle accident | BD-Gentest |
| 13* | 69 | M | Intracranial bleeding | In Vitro Technologies |
| * African-American Donors. All other donors were of Caucasian descent. 'N/A' - not available at time of purchase. | | | | |

| | | | | |
|---|---|---|---|---|
| Liver donor information reported here is specific information (age, sex, cause of death) on liver donors whose freshly isolated hepatocytes were purchased in suspension from multiple vendors for use in experiments of this study. | | | | |

Several other in vitro models of liver tissue have been proposed. In particular, multilayer or spheroid-based '3D' hepatocellular tissues, some with continuous perfusion, have been reported. As the liver itself is composed of flat, anastomising 'plates' that are typically one cell thick, two dimensional (monolayer) platforms of the liver may suffice for many ADME/Tox applications. Furthermore, since monolayer systems (confluent monolayers, collagen sandwich or Matrigel overlay) are still the most commonly utilized platforms in industry13,14, the microscale tissue proposed here can be mapped easily to existing laboratory protocols including robotic fluid handling, in situ microscopy, and colorimetric/fluorescent plate-reader assays.

A number of embodiments of the invention have been described. Nevertheless, it will be understood that various modifications may be made without departing from the spirit and scope of the invention. Accordingly, other embodiments are within the scope of the following claims.
Aspects and features of the present disclosure are set out in the following numbered clauses which contain the subject matter of the claims of the parent application as filed.
1. An *in vitro* cellular composition, comprising:
   (a) one or more populations of parenchymal cells defining a cellular island; and
   (b) a population of non-parenchymal cells,
   wherein the non-parenchymal cells define a geometric border of the cellular island.
2. The *in vitro* cellular composition of claim 1, wherein the parenchymal cells are selected from the group consisting of Hepatocytes, pancreatic cells (alpha, beta, gamma, delta), myocytes, enterocytes, renal epithelial cells, brain cells (neurons, astrocytes, glial cells), respiratory epithelial cells, adult and embryonic stem cells, and blood-brain barrier cells.
3. The *in vitro* cellular composition of claim 1, wherein the parenchymal cells are hepatocytes.
4. The *in vitro* cellular composition of claim 1, 2, or 3, wherein the non-parenchymal cells are stromal cells.
5. The *in vitro* cellular composition of claim 4, wherein the stromal cells are fibroblast cells or fibroblast derived cells .
6. The *in vitro* cellular composition of claim 1, wherein the cellular islands comprise a diameter or width of about 250µm to 750µm.
7. The *in vitro* cellular composition of claim 1, wherein the cellular islands are spaced apart from about 2µm to 1300 µm from center to center of the cellular islands.
8. The *in vitro* cellular composition of claim 1, located in a microfluidic device.
9. The *in vitro* cellular composition of claim 1, located in a tissue culture plate.
10. The *in vitro* cellular composition of claim 1, wherein the parenchymal cells are human cells.
11. The *in vitro* cellular composition of claim 1, wherein the non-parenchymal cells are human cells.
12. The *in vitro* cellular composition of claim 1, wherein the parenchymal and non-parenchymal cells are human cells.
13. The *in vitro* cellular composition of claim 1, wherein the cellular island is three-dimensional.
14. The *in vitro* cellular composition of claim 13, wherein the cellular island is a spheroid.
15. The *in vitro* cellular composition of claim 1, wherein the cellular island comprises parenchymal cells in a bounded geometry bordered by non-parenchymal cells.
16. A method of making a plurality of cellular islands on a substrate, comprising:
   (a) spotting an adherence material on a substrate at spatially different locations each spot having a defined geometric size and/or shape;
   (b) contacting the substrate with a population of cells that selectively adhere to the adherence material and/or substrate; and
   (c) culturing the cells on the substrate to generate a plurality of cellular islands.
17. The method of claim 16, wherein the spotting is performed by lithographic techniques.
18. The method of claim 17, wherein the lithographic technique is photolithography.
19. The method of claim 16, wherein the adherence material is selected from the group consisting of an extracellular matrix material, a sugar, a proteoglycan and any combination thereof.
20. The method of claim 16, wherein the population comprises a parenchymal cell population that selective adheres to the adherence material.
21. The method of claim 16, wherein the population comprises two or more cell types that selectively adhere to different locations or materials on the substrate.
22. The method of claim 20, wherein the parenchymal cell population is selected from the group consisting of hepatocytes, pancreatic cells (alpha, beta, gamma, delta), myocytes, enterocytes, renal epithelial cells, brain cells (neurons, astrocytes, glial cells), respiratory epithelial cells, adult and embryonic stem cells, and blood-brain barrier cells.
23. The method of claim 20, wherein the parenchymal cell population comprises hepatocytes.
24. The method of claim 20, further comprising contacting the substrate with a population that adheres to the substrate at a location different than the parenchymal cell population.
25. The method of claim 24, wherein the population comprises stromal cells.
26. The method of claim 25, wherein the stromal cells are fibroblast or fibroblast derived cells.
27. The method of claim 16, wherein the substrate is a tissue culture substrate.
28. The method of claim 16, wherein the substrate is glass or polystyrene.
29. The method of claim 16, wherein the defined diameter is about 250µm to 750µm.
30. The method of claim 16, wherein the spots are spatially separated by about 2µm to 1300 µm.
31. A cellular composition made by the method of claim 16.
32. An assay system comprising:
   contacting an artificial tissue comprising parenchymal cells having a bounded geometry bordered by non-parenchymal cells wherein the bounded geometry has at least one dimension from side to side of the bounded geometry of about 250µm to 750µm;
   contacting the artificial tissue with a test agent; and
   measuring an activity selected from gene expression, cell function, metabolic activity, morphology, and a combination thereof, of the artificial tissue.
33. The assay system of claim 32, wherein the test agent is selected from an infectious agent, a protein, a peptide, a polypeptide, an antibody, a peptidomimetic, a small molecule, an oligonucleotide, and a polynucleotide.
34. The assay system of claim 32, wherein the test agent is a cytotoxic agent.
35. The assay system of claim 32, wherein the test agent is a pharmaceutical agent.
36. The assay system of claim 32, wherein the test agent is a xenobiotic.
37. The assay system of claim 36, wherein the xenobiotic is selected from the group consisting of an environmental toxin, a chemical/biological warfare agent, a natural compound and a nutraceutical.
38. The assay system of claim 32, wherein the activity is adsorption, distributions, metabolism, excretion, and toxicology (ADMET) of the test agent.
39. The assay system of claim 32, wherein the metabolic activity is protein production.
40. The assay system of claim 32, wherein the metabolic activity is enzyme bioproduct formation.
41. The assay system of claim 32, wherein the parenchymal cells are human hepatocytes and the non-parenchymal cells are fibroblast.
42. An artificial tissue comprising islands of parenchymal cells surrounded by stromal cells wherein the islands of parenchymal cells are about 250µm to 750µm in diameter or width.
43. The artificial tissue of claim 42, wherein the parenchymal cells are human hepatocytes and the stromal cells are fibroblasts.
44. A method of producing a tissue *in* vitro, comprising:
   seeding a first population of cells on a substrate having defined regions for attachment of the first population of cells, wherein the defined regions comprise a bounded geometric dimension of about 250µm to 750µm;
   seeding a second population of cells on the substrate, such that the second population of cells surround or adhere adjacent to the first population of cells; and
   culturing the cells under conditions and for a sufficient period of time to generate a tissue.
45. The method of claim 44, wherein the first population of cells comprise human hepatocytes and the second population of cells comprise stromal cells.
46. The method of claim 45, wherein the stromal cells are fibroblasts.

## Claims

1. An *in vitro* cellular composition, comprising:
(a) one or more populations of parenchymal cells (optionally human cells) defining a cellular island; and
(b) a population of non-parenchymal cells (optionally human cells),
wherein the non-parenchymal cells define a geometric border of the cellular island.

2. The *in vitro* cellular composition of claim 1, wherein the parenchymal cells are selected from the group consisting of hepatocytes, pancreatic cells (alpha, beta, gamma, delta), myocytes, enterocytes, renal epithelial cells, brain cells (neurons, astrocytes, glial cells), respiratory epithelial cells, adult and embryonic stem cells, and blood-brain barrier cells and/or wherein the non-parenchymal cells are stromal cells, preferably fibroblast cells or fibroblast derived cells.

3. The *in vitro* cellular composition of claim 1, wherein the cellular islands comprise a diameter or width of about 250µm to 750µm and/or wherein the cellular islands are spaced apart from about 2µm to 1300µm from center to center of the cellular islands.

4. The *in vitro* cellular composition of claim 1, located in a microfluidic device or a tissue culture plate, or wherein the cellular island is three-dimensional, preferably a spheroid or wherein the cellular island comprises parenchymal cells in a bounded geometry bordered by non-parenchymal cells.

5. A method of making a plurality of cellular islands on a substrate, comprising:
(a) spotting an adherence material on a substrate at spatially different locations each spot having a defined geometric size and/or shape;
(b) contacting the substrate with a population of cells that selectively adhere to the adherence material and/or substrate; and
(c) culturing the cells on the substrate to generate a plurality of cellular islands.

6. The method of claim 5, wherein the spotting is performed by lithographic techniques, preferably photolithography or wherein the adherence material is selected from the group consisting of an extracellular matrix material, a sugar, a proteoglycan and any combination thereof or wherein the population comprises a parenchymal cell population that selectively adheres to the adherence material or wherein the population comprises two or more cell types that selectively adhere to different locations or materials on the substrate or wherein the substrate is a tissue culture substrate, preferably glass or polystyrene.

7. The method of claim 5, wherein the parenchymal cell population is selected from the group consisting of hepatocytes, pancreatic cells (alpha, beta, gamma, delta), myocytes, enterocytes, renal epithelial cells, brain cells (neurons, astrocytes, glial cells), respiratory epithelial cells, adult and embryonic stem cells, and blood-brain barrier cells, optionally further comprising contacting the substrate with a population that adheres to the substrate at a location different than the parenchymal cell population, optionally wherein the population comprises stromal cells, preferably fibroblast or fibroblast derived cells.

8. The method of claim 5, wherein the defined diameter is about 250µm to 750µm or wherein the spots are spatially separated by about 2µm to 1300µm.

9. A cellular composition made by the method of claim 5.

10. An assay system comprising:
contacting an artificial tissue comprising parenchymal cells having a bounded geometry bordered by non-parenchymal cells wherein the bounded geometry has at least one dimension from side to side of the bounded geometry of about 250µm to 750µm;
contacting the artificial tissue with a test agent; and measuring an activity selected from gene expression, cell function, metabolic activity, morphology, and a combination thereof, of the artificial tissue.

11. The assay system of claim 10, wherein the test agent is selected from an infectious agent, a protein, a peptide, a polypeptide, an antibody, a peptidomimetic, a small molecule, an oligonucleotide, and a polynucleotide, or wherein the test agent is a cytotoxic agent, a pharmaceutical agent or a xenobiotic.

12. The assay system of claim 11, wherein the xenobiotic is selected from the group consisting of an environmental toxin, a chemical/biological warfare agent, a natural compound and a nutraceutical.

13. The assay system of claim 10, wherein the activity is adsorption, distributions, metabolism, excretion, and toxicology (ADMET) of the test agent or wherein the metabolic activity is protein production or enzyme bioproduct formation or wherein the parenchymal cells are human hepatocytes and the non-parenchymal cells are fibroblasts.

14. An artificial tissue comprising islands of parenchymal cells surrounded by stromal cells wherein the islands of parenchymal cells are about 250µm to 750µm in diameter or width, optionally wherein the parenchymal cells are human hepatocytes and the stromal cells are fibroblasts.

15. A method of producing a tissue *in vitro,* comprising:
seeding a first population of cells on a substrate having defined regions for attachment of the first population of cells, wherein the defined regions comprise a bounded geometric dimension of about 250µm to 750µm;
seeding a second population of cells on the substrate, such that the second population of cells surround or adhere adjacent to the first population of cells; and
culturing the cells under conditions and for a sufficient period of time to generate a tissue, optionally wherein the first population of cells comprise human hepatocytes and the second population of cells comprise stromal cells, optionally wherein the stromal cells are fibroblasts.
